# EUROPÄISCHE PATENTSCHRIFT

(11) **EP 4 051 137 B1**
(45) Veröffentlichungstag und Bekanntmachung des Hinweises auf die Patenterteilung: **18.02.2026**
(21) Anmeldenummer: 20800085.1
(22) Anmeldetag: 29.10.2020
(51) Int. Cl.: A61B 17/16, A61B 17/32, A61B 90/70, A61B 18/14, A61B 18/00, A61B 90/00

(54) **CHIRURGISCHES INSTRUMENTENHANDSTÜCK**
SURGICAL INSTRUMENT HANDPIECE
PIÈCE À MAIN D'INSTRUMENT CHIRURGICAL

(30) Priorität: 29.10.2019 DE 102019129218
(43) Veröffentlichungstag der Anmeldung: 07.09.2022
(73) Patentinhaber: Aesculap AG, 78532 Tuttlingen (DE)
(72) Erfinder: HÖGERLE, Roland-Alois, 78532 Tuttlingen (DE); BÜRK, André, 78056 Villingen-Schwenningen (DE)
(74) Vertreter: Winter, Brandl - Partnerschaft mbB
(86) Internationale Anmeldenummer: PCT/EP2020/080346
(87) Internationale Veröffentlichungsnummer: WO 2021/083987

(56) Entgegenhaltungen:
- WO-A1-00/18521
- WO-A1-2006/094812
- WO-A1-2017/162786
- DE-A1- 102010 017 624
- DE-A1- 102017 108 272
- DE-U1- 202011 050 062
- US-A1- 2003 023 256
- US-A1- 2005 245 318
- US-A1- 2008 171 302
- US-A1- 2013 103 067
- US-A1- 2019 015 112
- US-A9- 2004 156 744

## Beschreibung

### Technisches Gebiet

Die vorliegende Offenbarung betrifft ein chirurgisches Instrumentenhandstück, wie sie zur Aufnahme bzw. zum Antrieb eines chirurgischen Werkzeugs im weitesten Sinne, insbesondere eines drehbaren Werkzeugs wie eines Fräsers, Bohrers, Schleifkopfs oder dergleichen, verwendet werden. Ferner betrifft die vorliegende Offenbarung ein solches chirurgisches Instrument sowie ein zugehöriges medizinisches Produktset mit dem chirurgischen Instrumentenhandstück in Kombination mit zumindest einem Zubehör. Desweiteren wird ein zugehöriges Reinigungsverfahren zur Innendurchspülung des chirurgischen Instrumentenhandstücks vorgeschlagen.

Im Stand der Technik der modernen minimalinvasiven Chirurgie, insbesondere der Neuro- und Wirbelsäulenchirurgie, ist es bekannt, chirurgische Instrumentenhandstücke zum Zwecke der Bearbeitung von bspw. Knochen, Knorpeln, Wirbeln, etc. einzusetzen. So betrifft die DE 10 2013 111 194 A1 der vorliegenden Anmelderin, die hiermit durch Verweis ausdrücklich zum Bestandteil der vorliegenden Offenlegungsschrift gemacht wird, ein gattungsgemässes chirurgisches Instrumentenhandstück. Dabei weist das jeweilige chirurgische Instrumentenhandstück üblicherweise zumindest eine Aufnahme bzw. Kupplung bzw. Kopplung für ein koppelbares, vorzugsweise drehbar antreibbares, Werkzeug, auf. Medizinische Indikationen, bei welchen chirurgische Instrumentenhandstücke, verwendet werden, umfassen Arthroskopien im Sinne der Untersuchung und/oder Behandlung von Gelenken, orthopädische Eingriffe, die Wirbelsäulenchirurgie, die Kieferchirurgie, die Neurochirurgie, usw.

Während der Verwendung der chirurgischen Instrumente (bzw. Instrumentenhandstücke) kommt das distale Ende des Instruments (bzw. des Instrumentenhandstücks) mit organischen und anorganischen Substanzen, wie bspw. Körperflüssigkeiten, Knochenabrieb, in Kontakt, die sich in Form von Ab- bzw. Anlagerungen am und/oder im Instrument (bzw. Instrumentenhandstück) festzusetzen neigen. Der Einsatz von wiederverwendbaren chirurgischen Instrumenten (bzw. Instrumentenhandstücken) erfordert deshalb vor und/oder nach jeder Benutzung eine sachgemäße Aufbereitung, insbesondere Reinigung und/oder Desinfektion, bei der die im und am Instrument anhaftenden Verschmutzungen zur abermaligen sterilen Verwendung des Instrumentes wieder entfernt werden.

Insbesondere die Reinigung der Lumen oder Hohlkörper des Instrumentenhandstücks mit seinen innenliegenden Flächen und/oder Teilen stellt eine besondere Herausforderung dar. Zu diesem Zweck wird das Instrumentenhandstück bzw. der Hohlkörper des Instrumentenhandstücks mit einem Reinigungsfluid, insb. einer Reinigungslösung, durchspült. Die Innendurchspülung mit dem Reinigungsfluid soll Schmutzpartikel, die sich an den innenliegenden Flächen und/oder Teilen des Hohlkörpers bzw. Instrumentenhandstücks angelagert haben, lösen und aus dem Hohlkörper bzw. Instrumentenhandstück austragen.

Dabei hat sich besonders die Reinigung der Zwischenräume im Hohlkörper des Instrumentenhandstücks, insbesondere seiner distalen (Patienten-zugewandten) Kugellager zur Aufnahme eines drehbar antreibbaren Werkzeugs wie eines Fräsers oder Bohrers, als schwierig erwiesen. Aufgrund des geringen Widerstands strömt das Reinigungsfluid beim Durchströmen des Instrumentenhandstücks fast ausschließlich durch die Innenringe bzw. entlang der Innenflächen der Innenringe der distalen Kugellager, sodass die Kugeln, Käfige und Zwischenräume dieser Kugellager einer geringeren Reinigungswirkung unterliegen.

Diese Problematik wird im Stand der Technik bereits aufgegriffen. So offenbart die Patentanmeldung der vorliegenden Anmelderin mit amtlich vergebenem Aktenzeichen DE 10 2018 133 503.2, deren Offenbarung hiermit durch Verweis ausdrücklich in die vorliegende Offenlegungsschrift einbezogen wird, eine separate Spülvorrichtung mit oder aus einem schaftartigen Spüleinsatz zur Innenreinigung eines Instrumentenhandstücks eines chirurgischen Instruments, insbesondere eine Spülvorrichtung zur Reinigung der distalen Kugellager im Innenraum eines Instrumentenhandstücks. Dabei wird die dort vorgeschlagene Spülvorrichtung in eine Werkzeugaufnahme bzw. in einen Werkzeugaufnahmeschacht des Instrumentenhandstückes nach dessen chirurgischer Verwendung zum Zwecke der Reinigung eingesetzt bzw. aufgesteckt.

Zwar überzeugt die vorstehend dargelegte Lösung aus dem Stand der Technik hinsichtlich der erzielbaren Reinigungseffizienz, jedoch besteht noch der Nachteil, dass mit der dort offenbarten Spülvorrichtung eine separate Vorrichtung, nämlich der schaftartige Spüleinsatz, zusätzlich zum Instrumentenhandstück in der Zentralen Sterilgutversorgungsabteilung (ZSVA) bzw. Zentralsterilisation bzw. Aufbereitungseinheit für Medizinprodukte (AEMP), vorgehalten und vor dem Reinigungszyklus in das Instrumentenhandstück eingesetzt werden muss. Es besteht die Gefahr, dass die separate Spülvorrichtung verlorengehen kann. Ferner muss die Handhabung und Anwendung der Spülvorrichtung gesondert beschrieben und umgesetzt werden.

Ferner noch bestehen, unabhängig vom oben diskutierten Reinigungsaspekt, weitere Nachteile für den Stand der Technik hinsichtlich chirurgischer Instrumentenhandstücke mit Blick auf deren medizinische, insb. chirurgische, Anwendung. Und zwar ergeben sich diese unter dem Gesichtspunkt der Außendimensionierung bzw. der Lichtmaße des Schaftabschnittes negativ. So besteht hinsichtlich minimalinvasiver, geringstmöglich traumatisierender Eingriffe ein Bedürfnis nach weitestgehend kleinen bzw. immer noch kleineren Dimensionierungen der chirurgischen Instrumentenhandstücke. So besteht zum einen bei der Sichtbarmachung des chirurgischen Eingriffs bzw. der Handhabung im Patienten in Echtzeit, insbesondere per endoskopischem Kamerabild mit Wiedergabe auf einem OP-Monitor für den Chirurgen, für die Instrumentenhandstücke im Stand der Technik der Nachteil, dass diese entsprechend ihrer Dimensionierung den Blick auf Gewebe versperren bzw. abschatten. Insbesondere im Bereich der Mikrochirurgie fällt dieses Sichthindernis umso vergrößerter und damit nachteiliger aus. Zum anderen besteht hinsichtlich des chirurgischen Zugangs, bspw. im Bereich der Hirnchirurgie, bzw. der operablen Indikationen als solchen ein Bedürfnis nach einer kleineren Dimensionierung der chirurgischen Instrumentenhandstücke bzw. nach einer demgemäßen Erweiterung des chirurgischen Einsatzfeldes.

US 2017 / 0 120 451 A1 offenbart ein chirurgisches Instrumentenhandstück in Form einer Baugruppe zum Halten eines Werkzeugs, wobei die Anordnung selektiv Vibrationen reduzieren und / oder beseitigen, die von einem Benutzer empfangen und gefühlt werden, kann. Durch die Reduzierung von Vibrationen kann das Rattern an einem Arbeitsende eines Werkzeugs verringert oder beseitigt werden. Dazu ist ein vibrationsdämpfendes mittleres Zwischenstück zwischen der einem Schaftabschnitt zur Werkzeugaufnahme und einem Griffabschnitt des chirurgischen Instrumentenhandstückes angeordnet. Dabei sind im Übergangsbereich des mittleren Zwischenstückes zwei zueinander geringfügig unterschiedliche Innendurchmesser offenbart, jedoch in einem proximal von einem Kugellager liegenden Abschnitt, somit den proximalen Schaftabschnitt innerhalb des Griffabschnitts betreffend. Zudem ist im Stand der Technik als potentiell problematisch zu sehen, dass der in einer Spülvorrichtung wie einem Reinigungs- und Desinfektionsgerät von extern an eine Anzahl mehrerer angeschlossener und zu reinigender Instrumentenhandstücke proximal vorgelegte gesamte Eingangsdruck des Reinigungsfluides (im Sinne einer Druckdifferenz bzw. eines Drucküberschüsses gegenüber dem atmosphärischen Umgebungsdruck) sich gemäß der Anzahl pro jeweiligem Instrumentenhandstück zu einem jeweiligen Spüldruck anteilig aufteilt und damit verringert. Demzufolge kann bei einer (zu) hohen Beladung der Spülvorrichtung im Stand der Technik der unzureichende Fall eintreten, dass der jeweilige Spüldruck einen im Sinne einer zuverlässigen und hinreichenden Fluidreinigung erforderlichen Mindestwert unterschreitet. Dieser technische Nachteil eines ggf. zu niedrigen jeweiligen Spüldrucks kann insbesondere in einer Situation mit hoher klinischer Auslastung auftreten.

Ferner noch kann im Stand der Technik die nachteilige Situation vorliegen, dass ein für die Durchströmung mit dem Reinigungsfluid zur Verfügung stehender freier Strömungsquerschnitt (bzw. ein inneres Leervolumen) des Instrumentenhandstückes entlang der Längsrichtung bzw. Strömungsrichtung von proximal nach distal erweitert. Dementsprechend verschlechtert sich die mechanische Reinigungswirkung von proximal nach distal, zusätzlich zu den allgemeinen Strömungsdruckverlusten insbesondere aufgrund der Rohrreibung und sonstiger Strömungswiderstandbeiwerte.

Somit liegt der Erfindung die Aufgabe zugrunde, ein chirurgisches Instrumentenhandstück für ein chirurgisches Instrument zu schaffen, welches die oben dargelegten Nachteile des Standes der Technik überwindet. Zunächst soll ein noch sicherer mittels Innendurchspülung reinigbares bzw. sterilisierbares Instrumentenhandstück (alternativ) ermöglicht werden. Insbesondere soll der konstruktive Aufbau des Instrumentenhandstücks eine gezielte und kraftvolle Reinigung der distalen Kugellager ermöglichen. Desweiteren besteht eine zusätzliche Aufgabe darin, dem Anwender ein weiteres Einsatzfeld operabler Indikationen bereitzustellen. Zudem besteht eine noch weitere Aufgabe darin, die Abläufe in der Zentralen Sterilgutversorgungsabteilung (ZSVA) zu vereinfachen, so dass diese konstengünstiger und weniger fehleranfällig ausfallen.

US 2003/023256A1 kann als nächstliegender Stand der Technik aufgefasst werden und offenbart ein chirurgisches Instrument zur Dissektion von Knochen und anderem Gewebe mit einer Spindel, einem Dissektionswerkzeug und einem zwischen der Spindel und dem Dissektionswerkzeug angeordneten Adapter. Die Spindel umfasst einen Hohlraum und ein männliches Element, das in den Hohlraum hineinragt. Der Adapter umfasst eine Antriebswelle, die sich entlang einer Achse erstreckt und ein erstes Ende mit einem allgemein zylindrischen Querschnitt und einer zentral angeordneten Öffnung aufweist, die sich teilweise entlang der Achse erstreckt. Das männliche Element wird von der Spindel getragen und erstreckt sich in die Öffnung des Präparationswerkzeugs. Der Hohlraum kann einen Antriebsabschnitt und eine Werkzeugaufnahmeöffnung definieren. Ein oder mehrere Ausrichtungsvorsprünge können sich vom Antriebsabschnitt in die Werkzeugaufnahmeöffnung erstrecken.

Ferner offenbart DE 20 2011 050062 U1 der vorliegenden Anmelderin ein gattungsgemäßes chirurgisches Instrument mit einem Schaft und einer im Schaft rotierbar gelagerten Antriebswelle, welche an ihrem distalen Ende ein Werkzeugelement trägt oder umfasst, wobei im distalen Endbereich des Schafts ein Radiallager angeordnet oder ausgebildet ist zum rotierbaren Lagern der Antriebswelle am Schaft, dadurch gekennzeichnet, dass das Radiallager (82) in Form eines Nadellagers (84) ausgebildet ist. Ferner betrifft die D2 ein chirurgisches Handstück mit einem im Gehäuse angeordneten Antrieb sowie zugehöriges chirurgisches Antriebssystem mit einer Steuer- und/oder Regelungsvorrichtung zum Steuern und/oder Regeln des Antriebs.

US 2013/103067A1 offenbart Gewebeentfernungsvorrichtungen, umfassend ein Handgehäuse, einen Motor und einen mit dem Handgehäuse en Gewebeentfernungsmechanismus. Der Gewebeentfernungsmechanismus kann ein röhrenförmiges Element, ein drehbares längliches Element, das in einem Lumen des röhrenförmigen Elements angeordnet ist, ein erstes Laufrad distal zu dem drehbaren länglichen Element und ein zweites Laufrad neben dem ersten Laufrad umfassen.

Weiterer Stand der Technik betrifft die Druckschriften US 2005/245318 A1, DE 10 2010 017624 A1, US 2004/156744 A9 bzw. WO 00/18521 A1, WO 2017/162786 A1 sowie WO 2006/094812 A1.

Die vorgenannten Aufgaben werden offenbarungsgemäß durch die Merkmale des gegen den nächstliegenden Stand der Technik, WO 2017/162786 A1, abgegrenzten Anspruchs 1 gelöst

Das chirurgische Instrumentenhandstück für ein chirurgisches Instrument als ein erster Aspekt der vorliegenden Offenbarung umfasst einen Griffabschnitt, welcher zur proximalen Handhabung durch einen Operateur dient, und einen Schaftabschnitt, welcher sich in distaler Längsrichtung vom Griffabschnitt erstreckt. Dabei ist ein Werkzeug an dem dem Griffabschnitt gegenüberliegenden, distalen Ende in einer distalen Austrittsöffnung des Schaftabschnitts angeordnet bzw. durch einen Nutzer wie dem Operateur oder einer OP-Assistenz anordbar. Unter einem Werkzeug im Sinne der Offenbarung ist jede Vorrichtung oder Einheit zu verstehen, mit der ein Operateur den Körper oder Körperteile eines Patienten oder Implantate oder dergleichen behandeln und/oder bearbeiten kann, wobei das Werkzeug mittels des Instrumentenhandstücks durch den Operateur geführt wird.

Offenbarungsgemäß weist der Schaftabschnitt im Bereich der distalen Austrittsöffnung zumindest einen verengten distalen Spitzenabschnitt auf.

Dabei bezieht sich der Begriff "verengt" bzw. "Verengung" auf eine Querschnittsfläche an dem distalen Ende, insbesondere auf ein lichtes Außenmaß des Schaftsabschnittes, vorzugsweise ferner noch auf einen inneren Strömungsquerschnitt.

Dabei ist im Sinne der vorliegenden Offenbarung unter dem Begriff "distal" die anwendungstechnische Perspektive des das offenbarungsgemäße Instrumentenhandstück handhabenden Operateurs bzw. Nutzers zu verstehen, was der Patienten-zugewandten Seite gleichkommt. Dementsprechend bezeichnet vorliegend der Begriff "proximal" die dem Operateur bzw. Nutzer zugewandte, also Patientenabgewandte Seite.

Also ist offenbarungsgemäß der Schaftabschnitt entlang seiner Längsrichtung in zumindest zwei (Längs-)Abschnitte, einen ersten Abschnitt und einen zweiten Abschnitt, von unterschiedlicher Querschnittsfläche unterteilt. Dabei wird der umfasste erste Abschnitt mit einer kleineren ersten Querschnittsfläche als verengter distaler Spitzenabschnitt des (gesamten) Schaftabschnitts bezeichnet. Und es wird der umfasste zweite Abschnitt mit einer, gegenüber dem ersten Abschnitt, größeren zweiten Querschnittsfläche als unverengter Abschnitt des (gesamten) Schaftabschnitts bezeichnet. Mit anderen Worten schliesst sich in distaler Längsrichtung an den Schaftabschnitt, welcher sich in distaler Längsrichtung vom Griffabschnitt erstreckt, bzw. an den unverengten zweiten Abschnitt des (gesamten) Schaftabschnitts der verengte erste Abschnitt bzw. der distale Spitzenabschnitt an. Sprich, eine erste Länge, entlang welcher sich der erste Abschnitt bzw. der distale Spitzenabschnitt in Form einer Verengung erstreckt, entspricht einem Teil der, als zweite Länge bezeichneten, Gesamtlänge des (gesamten) Schaftabschnitts. Also bezieht sich die Differenz aus der (gesamten) zweiten Länge und der ersten Länge auf den (übrigen) zweiten Abschnitt bzw. den unverengten Bereich des Schaftabschnitts.

Offenbarungsgemäß werden also aufgrund der distalen Strömungsquerschnittsverengung die Fließgeschwindigkeit des Reinigungsfluides und damit die hydrodynamische Reinigungswirkung erhöht.

Aufgrund des konstruktiven Aufbaus des offenbarungsgemäßen Instrumentenhandstücks wird ein Reinigungsfluid bei einer Innendurchspülung in einer Strömungsrichtung von proximal nach distal gezielt bis hin zur distalen Autrittsöffnung geleitet und somit nicht mehr größtenteils die im Stand der Technik vergleichsweise große und damit nur einen sehr geringen Strömungswiderstand bietende Öffnung eines Innenringes eines distalen Kugellagers weniger wirkungsvoll oder wirkungslos durchströmen. Mit anderen Worten dient die vorliegende Offenbarung einer Reduktion des Anteils nicht effektiv geleiteten bzw. sogar fehlgeleiteten Spülflusses des Reinigungsfluides. Dies wird unter weitreichender Beibehaltung eines für die Reinigungswirkung hinreichenden, idealerweise überschüssig hohen, Strömungsdruckes entlang der Längsachse des Instrumentenhandstückes als Hauptströmungsrichtung bewirkt.

Zudem verbessert die optimierte, offenbarungsgemäß im distalen Spitzenabschnitt verengte äußere Kontur des Schaftabschnitts vorteilhaft den Sichtzugang für den Operateur während eines Eingriffes.

Zusätzlich werden offenbarungsgemäß vereinfachte Abläufe in der Zentralen Sterilgutversorgungsabteilung (ZSVA) unterstützt, was sich positiv auf zum einen verringerte Betriebskosten sowie zum anderen die Qualitätssicherheit bzw. Zuverlässigkeit auswirkt. Die offenbarungsgemäße neue Bauweise der distalen Spitze, bzw. des vom Schaftabschnitt umfassten Spitzenabschnitts, des Instrumentenhandstücks erhöht die Reinigungswirkung, insbesondere hinsichtlich der distalen Kugellager und/oder hinsichtlich eines Innenflächenabschnitts des (distalen) Spitzenabschnitts, bei der manuellen oder maschinellen Reinigung, ohne dass dabei zusätzliche Produkte wie eine spezielle Spülvorrichtung eingesteckt werden müssen.

Es ist im Sinne der Offenbarung nicht relevant, ob zusätzlich zu dem zumindest einen verengten distalen Spitzenabschnitt noch weitere Abschnitte von einer weiteren unterschiedlichen Querschnittsfläche von dem Schaftabschnitt, insb. von dem ersten Abschnitt bzw. dem distalen Spitzenabschnitt selber, umfasst sind. Mit anderen Worten ist es denkbar, dass weitere umlaufende Schaftabsätze und/oder Schaftstufen ausgebildet sind.

Desweiteren ist es im Sinne der Offenbarung nicht relevant, welcher Art ein, vorzugsweise am proximalen Ende des Griffabschnittes angeordneter, Anschluss bzw. eine Schnittstelle des Instrumentenhandstückes zur externen Energieversorgung bzw. zur Handhabung bzw. zum Antrieb des, vorzugsweise drehbar antreibbaren, Werkzeuges ausgestaltet ist. Je nach Verwendungszweck und beabsichtigter Werkzeugdrehzahl kann ein hydraulischer, pneumatischer und/oder elektromotorischer Antrieb vorgesehen bzw. wirkverbunden sein.

Ferner ist im Sinne der Offenbarung nicht relevant, dass die Verengung bzw. die verengte Querschnittsfläche eine spezifische Form aufweisen. Diese Begriffe sind vorliegend also nicht nur auf längliche Formen mit einem konstanten runden Querschnitt beschränkt aufzufassen, derart dass sich radial umlaufende Schaftabsätze und/oder radiale Stufen ausbilden. Hingegen umfasst die vorliegende Terminologie auch jegliche Verengungen mit einem entlang ihrer Längsache veränderlichen Querschnitt und/oder mit einem Querschnitt, welcher eine unrunde Form, bspw. eine ovale, rechteckige, konvexe und/oder konkave Form, aufweist. Insbesondere kann die Verengung nur in einem Winkelsegment ausgebildet sein bzw. eine asymmetrisch verengte Querschnittsfläche ausbilden.

Die Unteransprüche zeigen bevorzugte Weiterbildungen der Erfindung.

Häufig jedoch mögen aus produktionstechnischen Gründen wie auch unter anwendungstechnischen, insb. strömungsdynamischen, Gesichtspunkten radial konstante bzw. runde Querschnittsflächen bzw. Verengungen des Schaftabschnitts bevorzugt sein.

Somit ist es unter letztgenannten Gesichtspunkten bevorzugt, dass das chirurgische Instrumentenhandstück derart weitergebildet ist, dass ein erster Durchmesser des verengten distalen Spitzenabschnitts gegenüber einem zweiten Durchmesser eines unverengten Bereichs des Schaftabschnitts um einen Durchmesser-Relationsfaktor von maximal 95 Prozent, vorzugsweise von maximal 85 Prozent, weiter bevorzugt von ca. 79 Prozent kleiner ist.

Alternativ oder kumulativ beträgt dabei vorzugsweise der erste Durchmesser zwischen 3,5 und 5,3 Millimeter, vorzugsweise zwischen 4,0 und 5,0 Millimeter, weiter bevorzugt zwischen 4,3 und 4,5 Millimeter.

Dabei kann insbesondere bevorzugt eine Durchmesseränderung bzw. Verengung vom zweiten Durchmesser wie einem Außendurchmesser des Schaftabschnitts von ca. 5,6 mm, insbesondere bezogen auf einen an den Griffabschnitt anliegenden Bereich, hinunter auf ca. 4,4 mm für den ersten Durchmesser, wie einem Außendurchmesser des verengten distalen Spitzenabschnitts, vorgesehen sein.

Vorzugsweise ist dabei das chirurgische Instrumentenhandstück derart weitergebildet, dass eine erste Länge des distalen Spitzenabschnitts zwischen 5 und 40 Millimeter, vorzugsweise zwischen 10 und 30 Millimeter, weiter bevorzugt zwischen 18 und 22 Millimeter beträgt.

Alternativ oder kumulativ bemisst dabei vorzugsweise die erste Länge, insoweit sie auf eine, gesamte, zweite Länge des Schaftabschnitts bezogen bzw. in Relation gesetzt bzw. normiert wird, einen prozentualen Längenanteil von mindestens 5 Prozent, vorzugsweise von mindestens 20 Prozent, weiter bevorzugt von mindestens 35 Prozent.

Insbesondere kann ein Instrumentenhandstück bevorzugt sein, bei welchem sich der, vorzugsweise ca. 4,4 mm breite, distale Spitzenabschnitt auf ca. 20 mm als erste Länge erstreckt, während die zweite (gesamte) Länge gemäß einer anwendungstechnisch bzw. chirurgisch-operativ optimalen Länge für den, vorzugsweise ca. 5,6 mm breiten, (gesamten) Schaftabschnitt gewählt bzw. gesetzt ist.

Dazu konnte experimentell anhand von beispielhaften Prototypen gefunden werden, dass solche besonders bevorzugte Ausführungsformen der Offenbarung eine weiter optimierte Balance aller anwendungstechnischen Dimensionen bzw. unabhängiger Gruppen technischer Problematiken bzw. Aufgabenstellungen darstellen. So betreffen nämlich die vorstehend genannten chirurgisch-operativen Gesichtspunkte eine erste Gruppe von technischen Aufgaben und die strömungsdynamischen Effekte im Sinne bzw. zum Zwecke der nachfolgenden Reinigung eine zweite Gruppe von technischen Aufgaben. Insofern betrifft die erste Gruppe einen ersten Anwendungszeitraum während des chirurgischen bzw. operativen Einsatzes, insbesondere durch einen Operateur als einen ersten Anwender; und es betrifft die zweite Gruppe einen zweiten Anwendungszeitraum nach dem erfolgten chirurgischen bzw. operativen Einsatz, insbesondere durch Personal im Tätigkeitsbereich der Reinigung bzw. Sterilisation von OP-Besteck als einem zweiten Anwender.

Allerdings versteht sich, dass die Offenbarung nicht auf die vorgenannten besonders bevorzugten Ausführungsformen zu beschränken ist. Nicht zuletzt im Falle einer Miniaturisierung - wie aus den wissenschaftlichen Grundlagen der Kennzahlen-Problematik der Strömungsdynamik vorbekannt - sind abweichende bzw. andere absolute und/oder relative Dimensionierungen zu wählen bzw. bevorzugt.

Vorzugsweise ist ein als Absatz von dem verengten distalen Spitzenabschnitt zu dem unverengten Bereich des Schaftabschnitts ausgebildeter Übergangsbereich gerundet und/oder gradiell auslaufend und/oder angeschrägt geformt. Eine derartige Vermeidung eines kantigen bzw. sprunghaften Übergang zwischen dem ersten Abschnitt und dem zweiten Abschnitt bietet den Vorteil einer geringeren Schmutzanhaftung bzw. einer geringeren Gewebetraumatisierung bei der operativen Handhabung bzw. bei der Einführung in vom Chirurgen eröffnetes Gewebe.

Vorzugsweise ist das chirurgische Instrumentenhandstück dazu ausgelegt, in eine Spülvorrichtung wie in ein Reinigungs- und Desinfektionsgerät eingesetzt zu werden, derart dass der Spüldruck optimiert ist, welcher bei einer Innendurchspülung des Instrumentenhandstücks mit einem Reinigungsfluid, vorzugsweise mit einer hydrophilen oder lipophilen Reinigungslösung, in Strömungsrichtung von proximal nach distal an der distalen Austrittsöffnung anliegt. Dazu beträgt der zuvor genannte Spüldruck größer als 10 mbar, weiter bevorzugt größer als 90 mbar, noch weiter bevorzugt größer 160 mbar und insbesondere größer 500 mbar. Alternativ oder kumulativ bleibt der Spüldruck, insofern dieser auf einen proximal vorgelegten Eingangsdruck des Reinigungsfluids bezogen bzw. normiert wird, zu einem Anteil von mindestens 20 Prozent, vorzugsweise von mindestens 50 Prozent, weiter bevorzugt von mindestens 80 Prozent aufrechterhalten. Auf diese Weise kann eine besonders hohe Reinigungswirkung erzielt werden, wie insbesondere anhand von standardisierten Reinigungsprüfwerten belegbar, wie sie in der komplexen Fachkunde und den gesetzlichen Richtlinien für die Aufbereitung von Medizinprodukten Verwendung finden.

Dies ist besonders vorteilhaft, um der im Stand der Technik auftretenden Problematik eines sich entlang der Längsrichtung des Instrumentenhandstücks von proximal zu distal bzw. entlang der Strömungsrichtung (bzw. entlang der Richtung der Stromlinien), teils erheblich, reduzierenden Spüldrucks bzw. Strömungsdrucks effektiv entgegenzutreten. Diese Problematik bei herkömmlichen Instrumentenhandstücken kann besonders gravierend ausfallen, wenn bzw. insoweit die (innere) Strömungsquerschittsfläche nicht nur nicht konstant ist, sondern sogar sich entlang der Längsrichtung des Instrumentenhandstücks von proximal zu distal bzw. entlang der Strömungsrichtung erhöht, insbesondere sogar signifikant erhöht. Die Kontinuitätsgleichung für die (inkompressible) Strömung besagt A·v = V = const.; wobei A die (innere) Strömungsquerschittsfläche; v eine (gemittelte) Durchflussgeschwindigkeit des Reinigungsfluids; und V einen (inneren) Volumenstrom des Reinigungsfluids bezeichnet. Damit geht gemäß der Kontinuitätsgleichung für die (inkompressible) Strömung einher, dass die (negativ zu der Strömungsquerschittsfläche korrelierende) Durchflussgeschwindigkeit entsprechend abfällt. Demzufolge sinkt der Strömungsterm für die kinetische Energie (vgl. Bernoulli-Gleichung), wodurch gleichermaßen eine mechanische Reinigungswirkung in ungünstiger Weise im Stand der Technik abfällt.

Vorzugsweise kann eine Reynoldszahl bzw. Re für die Durchströmung in dem verengten distalen Spitzabschnitt einen turbulenten Bereich markieren, insbesondere oberhalb Re = 2300. Dabei sind in die Reynoldszahl als eine Dichte des Fluids und als eine dynamische Viskosität des Fluids diejenigen Stoffwerte des Reinigungsfluids, insbesondere von Wasser, einzusetzen. Als eine Strömungsgeschwindigkeit des Fluids ist ferner die (gemittelte) Durchflussgeschwindigkeit des Reinigungsfluids v heranzuziehen. Dabei wird vorliegend als eine charakteristische Länge des Körpers (bzw. des Rohres), auch Bezugslänge genannt, vorzugsweise ein Durchmesser eines der Strömung zur Verfügung stehenden (Strömungs-)Querschnitts, weiter bevorzugt ein Durchmesser des Innenflächenabschnitts des (distalen) Spitzenabschnitts definiert. Ein turbulenter Bereich kann Vorteile hinsichtlich einer besonders kraftvollen Reinigung im Falle hartnäckigster Anschmutzungen bzw. Verunreinigungen bewirken.

Alternativ zu einer einen turbulenten Bereich in dem verengten distalen Spitzabschnitt markierenden Reynoldszahl, kann es gerade bevorzugt sein, dass die Reynoldszahl einen laminaren Bereich in dem verengten distalen Spitzabschnitt markiert. Besonders bevorzugt kann Re zwischen 1000 und 2000 liegen. Das hat den Vorteil einer gleichmäßigen Durchströmung des offenbarungsgemäßen Instrumentenhandstückes unter Vermeidung von Pulsation und/oder Fluid-Wand-Wechselwirkungen. Das ermöglicht einen besonders gleichmäßigen, geräuscharmen bzw. vibrationsarmen Reinigungsbetrieb einer Spülvorrichtung.

Vorzugsweise umfasst der Schaftabschnitt eine innere Wälzlagerung zur drehbaren Lagerung des angeordneten oder anordbaren Werkzeuges, vorzugsweise zumindest teilweise im Bereich des verengten distalen Spitzenabschnitts. Dabei umfasst die Wälzlagerung zumindest ein distales Wälzlager und zumindest ein proximales Wälzlager. Zudem sind dabei das zumindest eine distale Wälzlager und das zumindest eine proximale Wälzlager durch einen zwischen ihnen durchgehend ausgebildeten Lagerkäfig beabstandet angeordnet.

Aufgrunddessen wird offenbarungsgemäß das im Stand der Technik verzeichnete nachteilige Strömungsverhalten im Vorfeld vermieden, welches ein Strömungsmaximum der Durchströmung entlang einer Zylinderachse des Schaftabschnitts bzw. im Mittelpunkt der zweiten Querschnittsfläche aufweist. Dabei ergibt sich dieses im Stand der Technik nachteilige Strömungsverhalten aus dem Strömungsgesetz des Weges gemäß dem geringsten Strömungswiderstand und/oder gemäß der als Wandhaftungsbedingung bekannten Strömungsbedingung, insb. im Falle einer Rohrströmung, und/oder ferner noch für die Betrachtung eines Inneren einer Wälzlagerung gemäß der Durchströmung einer Schüttung ("Pre-Darcy"). Mit anderen Worten, folgert im Stand der Technik eine nachteilige hydrodynamische Verteilung der Durchflussgeschwindigkeit (bzw. des Vektoranteils in der Längsrichtung des Instrumentenhandstückes von proximal nach distal) mit einem Maximum im Zentrum des Schaftabschnitts bzw. entlang einer Rotationsachse des Instrumentenhandstückes.

Insbesondere kann eine gesamte Länge des durchgehend ausgebildeten Lagerkäfigs mindestens 90 % der ersten Länge des distalen Spitzenabschnitts betragen. Alternativ oder kumulativ kann eine dem durchgehend ausgebildeten Lagerkäfig zugehörige Abstandslänge zwischen dem zumindest einen proximalen Wälzlager, bspw. (aber nicht limitierend) von einer Mittellinie davon, und dem zumindest einen distalen Wälzlager, bspw. (aber nicht limitierend) von einer Mittellinie davon, mindestens 60 %, weiter bevorzugt mindestens 70 % und insbesondere mindestens 78 % der ersten Länge betragen.

Die insbesondere bevorzugte Ausführungsform der vorliegenden Offenbarung betreffs eines durchgehend ausgebildeten Lagerkäfigs überwindet den vorstehend beschriebenen Nachteil aus dem Stand der Technik in besonders effektiver Weise. Insofern wird eine Zwangsdurchströmung durch die innere Wälzlagerung und/oder entlang des Innenflächenabschnitts bewirkt. Somit wird die mechanische Reinigungswirkung mittels des Reinigungsfluides in dem an die Spülvorrichtung, insbesondere an das Reinigungs- und Desinfektionsgerät, angeschlossenen Zustand weiter intensiviert. Infolgedessen findet eine zuverlässige und volle Fluidreinigung genau an den Stellen bzw. Oberflächen statt, und dies auch wirkungsvoll, an welchen Verunreinigungen anhaften können. Mit anderen Worten wird die Effizienz der Fluidreinigung bzw. der Durchspülung erhöht, indem vermieden wird, dass sich das Reinigungsfluid den Weg des geringsten (Strömung-)Widerstandes sucht und mittig entweicht. Anstattdessen wird offenbarungsgemäß das Reinigungsfluid gezielt in Richtung der zu reinigenden Oberflächen gelenkt, wo es die mechanische Reinigungswirkung entfalten soll, nämlich insbesondere innerhalb des zumindest einen distalen Wälzlagers und zumindest einen proximalen Wälzlagers und/oder entlang des Innenflächenabschnitts des verengten distalen Spitzenabschnitts.

Vorliegend sind als Wälzlager(ungen) solche Lager aufzufassen, bei denen zwischen einem Innenring und einem Außenring, im Gegensatz zu der Schmierung in Gleitlagern, rollende Wälzkörper wie bspw. Kugeln, Zylinder, Nadeln, Tonnen oder Kegel, den Reibungswiderstand verringern.

Bei der Ausführungsform mit der inneren Wälzlagerung, vorzugsweise mit dem durchgehend ausgebildeten Lagerkäfig, wird zusätzlich eine gezielte Strömungsführung bzw. eine Zwangsdurchströmung des Reinigungsfluids durch ein Spaltleervolumen, welches zwischen einem Innenflächenabschnitt des (distalen) Spitzenabschnitts und einem Lagerkäfig-Außenflächenabschnitt entsteht bzw. ausgebildet wird, bewirkt.

Darüber hinaus kann vorzugsweise im Gegensatz zum Stand der Technik eine Situation bevorzugt sein, bei welcher (nahezu) alle (mit Ausnahme des Spaltleervolumens) sonstigen etwaigen Strömungspfade des Instrumentenhandstückes wie eine Innenbohrung durch zumindest ein komplementär eingerichtetes Bauteil wie eine Antriebwelle verschlossen sind. Mit anderen Worten, findet die Zwangsdurchströmung ausnahmslos (bzw. vollständig) durch das Spaltleervolumen statt.

Insbesondere kann das Spaltleervolumen ein Ringspaltleervolumen betreffen. Insofern können weiter bevorzugt der Innenflächenabschnitt des (distalen) Spitzenabschnitts und der Lagerkäfig-Außenflächenabschnitt zylindrisch ausgebildet sein, weiter bevorzugt konzentrisch, d.h. auf eine zusammenfallende Mittenachse, angeordnet sein. Dabei ist es für die vorliegende Offenbarung unerheblich, ob bzw. inwieweit, insbesondere fertigungstechnische, Toleranzen bzw. Passungen vorliegen. Insbesondere können letztere alternativ oder kumulativ eingerichtet sein, ein Auslegungs- bzw. Konstruktionskriterium und/oder ein Betriebskriterium, bevorzugt eine Rundlaufeigenschaft der inneren Wälzlagerung und/oder einen maschinendynamischen Parameter, zu berücksichtigen.

Dem (jeweiligen) (Ring-)Spaltleervolumen entspricht eine zugehörige (jeweilige Strömungs-) Querschnittsfläche. So ist eine zylindrische Ringspalt-Querschnittsfläche zwischen einem äußeren Ringspalt-Außendurchmesser (eines zylindrischen Innenbauteils, insbesondere des durchgehend ausgebildeten Lagerkäfigs) und einem inneren Ringspalt-Innendurchmesser zur Zwangsdurchströmung ausgebildet. Somit berechnet sich die Ringspalt-Querschnittsfläche als eine (jeweilige Strömungs-)Querschnittsfläche aus der Subtraktion der beiden Kreisflächen mit dem Ringspalt-Außendurchmesser bzw. mit dem Ringspalt-Innendurchmesser jeweils.

Besonders bevorzugt kann die (jeweilige Strömungs-)Querschnittsfläche, insbesondere Ringspalt-Querschnittsfläche, in Strömungsrichtung des Reinigungsfluids bzw. entlang der zweiten Länge des gesamten Schaftabschnittes (von proximal nach distal), insbesondere von einem proximalen Bereich im Übergang zu dem Griffabschnitt bis hin zu einem distalen Bereich an dem proximalen Wälzlager und/oder zu der Austritts-Querschnittsfläche nicht zunehmen. Mit anderen Worten, kann die insbesondere Ringspalt-Querschnittsfläche in Strömungsrichtung konstant bleiben und/oder sich verengen. Dabei ist es weiter bevorzugt, dass die Verengung stetig verläuft bzw. keine Unstetigkeitsstellen aufweist. Das dient dazu, eine sprunghafte Veränderung des Strömungszustandes zu vermeiden. Insbesondere werden auf diese Weise lokale Totzonen bzw. Verwirbelungen bzw. Drosseleffekte vermieden, welche lokale Verschlechterungen der mechanischen Fluidreinigung nach sich ziehen können.

Bei der bevorzugten Ausführungsform mit der inneren Wälzlagerung in Weise des durchgehend ausgebildeten Lagerkäfigs ergibt sich die, um den äußeren Umfang des (mittigen) Lagerkäfigs ausgebildete, Lagerkäfig-Ringspalt-Querschnittsfläche aus der Subtraktion der beiden Kreisflächen mit dem Innenflächenabschnitt-Durchmesser als dem Ringspalt-Außendurchmesser bzw. mit dem Lagerkäfig-Außendurchmesser als dem Ringspalt-Innendurchmesser jeweils.

Vorzugsweise kann die Lagerkäfig-Ringspalt-Querschnittsfläche im distalen verengten Spitzenabschnitt kleiner-gleich 3,5 mm², weiter bevorzugt kleiner-gleich ca. 3 mm² und insbesondere bevorzugt kleiner-gleich 2,8 mm² betragen.

Alternativ oder kumulativ vorzugsweise kann die Lagerkäfig-Ringspalt-Querschnittsfläche im distalen verengten Spitzenabschnitt kleiner-gleich einer in einem dazu proximalen Bereich durchströmbaren Strömungsquerschnittsfläche, insbesondere kleiner-gleich einer proximalen Schaftabschnitt-Ringspalt-Querschnittsfläche, sein. Dabei kann die proximale Schaftabschnitt-Ringspalt-Querschnittsfläche den zu dem Schaftabschnitt proximalen Bereich im Übergang zu dem Griffabschnitt und/oder einen zu dem proximalen Wälzlager proximalen Bereich der Werkzeugaufnahme im Schaftabschnitt betreffen.

Dabei kann insbesondere die (distale) Lagerkäfig-Ringspalt-Querschnittsfläche kleiner-gleich 85 %, weiter bevorzugt kleiner-gleich 80 % und insbesondere bevorzugt kleiner-gleich 76,5 % der proximalen Schaftabschnitt-Ringspalt-Querschnittsfläche betragen.

Alternativ oder kumulativ kann dabei insbesondere die (distale) Lagerkäfig-Ringspalt-Querschnittsfläche kleiner-gleich 150 %, weiter bevorzugt kleiner-gleich 130 % und insbesondere bevorzugt kleiner-gleich ca. 122 % einer (proximalen) Instrumentenhandstück-Eingangs-Querschnittsfläche betragen. Dabei betrifft die Instrumentenhandstück-Eingangs-Querschnittsfläche eine im Anschlussbereich an die Spülvorrichtung angeordnete (frei) durchströmbare Strömungsquerschnittsfläche des Instrumentenhandstückes. Insbesondere bezeichnet die Instrumentenhandstück-Eingangs-Querschnittsfläche ein Minimum der Strömungsquerschnittsfläche bzw. einen Engpass, wenn auf die Durchströmung des gesamten Instrumentenhandstückes bezogen.

Besonders bevorzugt ist die Durchflussmenge bzw. der Volumenstrom des Reinigungsfluids und/oder die Durchflussgeschwindigkeit des Reinigungsfluids durch das zumindest eine distale Wälzlager und/oder das zumindest eine proximale Wälzlager erhöht. Insbesondere ist ein Längsvektoranteil der Durchflussgeschwindigkeit in der Längsrichtung des Schaftabschnittes bzw. in der Längsrichtung des Spitzenabschnittes erhöht. Daraus ergibt sich der Vorteil, dass Zonen mit einer hinsichtlich einer mechanischen Reinigungswirkung nur unzureichend hohen Durchflussgeschwindigkeit des Reinigungsfluids und/oder Totzonen vermieden werden.

Die Erhöhung der Durchflussgeschwindigkeit, insbesondere des Längsvektoranteils, durch die innere Wälzlagerung bzw. entlang der inneren Wälzlagerung kann insbesondere gemäß einen jeweilig zugehörigen Lagerkäfig-Intensivierungsfaktor bemessen bzw. quantifiziert werden:
Zum einen kann sich der jeweilig zugehörige Lagerkäfig-Intensivierungsfaktor, was einen primären bzw. ersten Lagerkäfig-Intensivierungsfaktor betrifft, im Vergleich bzw. in Relation einer offenbarungsgemäßen Situation, d.h. mit einem verengten distalen Spitzenabschnitt, zu einer bzw. bezogen auf eine herkömmliche(n) Situation nach dem einleitend beschriebenen Stand, d.h. ohne Vorhandensein eines verengten distalen Spitzenabschnittes, bemessen.

Vorzugsweise kann der erste Lagerkäfig-Intensivierungsfaktor, insbesondere bezogen auf das distale Wälzlager, größer-gleich 1,5, weiter bevorzugt größer-gleich 2,5 und insbesondere bevorzugt größer-gleich 3 betragen. Insofern in die Berechnung eines Terms der kinetischen Energie einer Strömung die Durchflussgeschwindigkeit zu einem quadratischen Exponenten eingeht, bewirkt eine Erhöhung des ersten Lagerkäfig-Intensivierungsfaktors eine spürbare Erhöhung der kinetischen Energie und somit der mechanischen Reinigungsleistung. Insbesondere wird positiv bewirkt, dass alle Zonen bzw. zu reinigenden Oberflächen zuverlässig umspült und kraftvoll fluidgereinigt werden.

Zum anderen kann sich der jeweilig zugehörige Lagerkäfig-Intensivierungsfaktor, was einen sekundären bzw. zweiten Lagerkäfig-Intensivierungsfaktor betrifft, im Vergleich bzw. in Relation zu einer offenbarungsgemäßen Situation, also mit einem verengten distalen Spitzenabschnitt bemessen. Demzufolge gibt der zweite Lagerkäfig-Intensivierungsfaktor für die weiter bevorzugte Ausführungsform mit dem durchgehend ausgebildeten Lagerkäfig an, wie dieser die offenbarungsgemäße Situation mit einem verengten distalen Spitzenabschnitt (ohne den durchgehend ausgebildeten Lagerkäfig) noch weiter verbessert. Vorzugsweise kann der zweite Lagerkäfig-Intensivierungsfaktor, insbesondere bezogen auf das distale Wälzlager, größer-gleich 1,1, weiter bevorzugt größer-gleich 1,5 und insbesondere bevorzugt größer-gleich 2 betragen.

Bei der bevorzugten Ausführungsform mit der inneren Wälzlagerung in Weise des durchgehend ausgebildeten Lagerkäfigs wird als die Bezugslänge für die Reynoldszahl eine Breite des Lagerkäfig-Ringspalts definiert. Dabei ermittelt sich die Breite des Lagerkäfig-Ringspalts aus der Subtraktion des Lagerkäfig-Außendurchmessers als dem Ringspalt-Innendurchmesser von dem Innenflächenabschnitt-Durchmesser als dem Ringspalt-Außendurchmesser. Ansonsten wird auf vorstehend zu der Reynoldszahl Offenbartes verwiesen. In der bevorzugten Ausführungsform ist von besonderem Vorteil, dass die Reynoldszahl abgesenkt wird. Insbesondere kann hinsichtlich der Durchströmung ein ansonsten (beginnend) turbulenter Bereich durch Einsetzen des Lagerkäfigs und damit einer dementsprechenden Reduktion der Bezugslänge in einen laminaren Bereich umgekehrt werden.

Insgesamt werden demgemäß bei der Ausführungsform mit der inneren Wälzlagerung, vorzugsweise mit dem durchgehend ausgebildeten Lagerkäfig, Vorteile dahingehend erzielt, dass die Reinigungswirkung beim inneren Durchspülen des Instrumentenhandstücks noch weiter verbessert wird, indem eine gezielte Strömungsführung des Reinigungsfluids stattfindet. Insbesondere findet eine Zwangsdurchströmung bzw. Zwangskonvektion des Reinigungsfluids durch das zumindest eine distale Wälzlager und/oder das zumindest eine proximale Wälzlager statt, was ein intensives Umspülen der Wälzkörper sicherstellt. Infolgedessen kommt es zu einer bei Vorhandensein des durchgehend ausgebildeten Lagerkäfigs sogar noch weiter verstärkten hydromechanischen Wirkung. Damit gelingt auch bei einem solchen Fluidreinigungsbetrieb der Spülvorrichtung, insbesondere dem Reinigungs- und Desinfektionsgerät, welcher mit einer hohen Anzahl an den angeschlossenen Instrumentenhandstücken, d.h. an besonders vielen, insbesondere (nahezu) allen darin vorgesehenen Eingangs-Anschlüssen bzw. Adaptern bzw. Flanschen beaufschlagt ist, eine zuverlässige Fluidreinigung. Sprich, auch bei kleinen Teilvolumenströmen, in welche sich ein der Spülvorrichtung zur Verfügung stehender Gesamtvolumenstrom des Reinigungsfluides gemäß der Anzahl der angeschlossenen Instrumentenhandstücke aufteilt bzw. splittet, ist eine ausreichende Fluiddurchströmung und infolgedessen eine ausreichend starke mechanische Reinigungswirkung gewährleistet.

Wie oben bereits ausgeführt, ist das chirurgische Instrumentenhandstück dazu ausgelegt, in eine Spülvorrichtung wie in ein Reinigungs- und Desinfektionsgerät eingesetzt zu werden, um eine Innendurchspülung des Instrumentenhandstücks mit einem Reinigungsfluid, vorzugsweise mit einer hydrophilen oder lipophilen Reinigungslösung, in Strömungsrichtung von proximal nach distal durchführen zu können. Im Falle des direkt zuvor beschriebenen bevorzugten Ausführungsform des chirurgischen Instrumentenhandstücks (mit insb. einem durchgehend ausgebildeten Lagerkäfig) beträgt der dann an der distalen Austrittsöffnung anliegende Spüldruck vorzugsweise größer 600 mbar, vorzugsweise größer 700 mbar und weiter bevorzugt ca. 800 mbar. Somit stellt diese besonders bevorzugte Ausführungsform in höchstem Maße eine effektive Reinigung sicher.

Vorzugsweise ist der Lagerkäfig vollständig geschlossen ausgeführt. Das hat den Vorteil einer maximalen Reinigungskraft für die distalen Wälzlager. Alternativ ist der Lagerkäfig zu einem geringen flächenbezogenen Lochvolumenanteil fluiddurchlässig. Dabei kann vorzugsweise der Lochvolumenanteil kleiner 40 Prozent, weiter bevorzugt kleiner 15 Prozent, insbesondere bevorzugt kleiner 8 Prozent betragen. Dies erlaubt eine weitere Optimierung im Sinne einer möglichst gleichmäßigen Reinigungswirkung auf Basis einer über den Lagerkäfig hinweg verfeinerten Strömungsführung. Letztere kann bspw. mittels einer fluiddynamischen Modellierung bzw. mittels Berechnungsmethoden anhand finiter Volumenelemente des inneren Strömungsraumes des Instrumentenhandstückes erzielt werden.

Vorzugsweise weisen das zumindest eine distale Wälzlager und/oder das zumindest eine proximale Wälzlager, vorzugsweise alle Wälzlager der inneren Wälzlagerung, nicht-kugelige Wälzkörper auf. Dabei ist es weiter bevorzugt, dass das zumindest eine distale Wälzlager und/oder das zumindest eine proximale Wälzlager als Zylinderrollenlager und/oder als Nadellager ausgebildet ist. Die Wälzkörper von Zylinderrollenlagern sind Kreiszylinder. Zylinderrollenlager werden in unterschiedlichen Bauformen gefertigt, wie in der DIN-Norm 5412 beschrieben, deren Offenbarung durch Verweis miteinbezogen wird. Ein Nadellager hat kreiszylindrische Wälzkörper, als Nadeln bezeichnet, wobei diese sehr große Längen im Verhältnis zum Wälzkörperdurchmesser (Verhältnisfaktor größer-gleich ca. 2,5) aufweisen. Nadellager sind in der DIN-Norm 617 normiert, deren Offenbarung durch Verweis miteinbezogen wird. Bei diesen bevorzugten Ausführungsformen mit nicht-kugeligen Wälzkörpern zeichnet sich die Wälzlagerung durch eine große radiale Tragfähigkeit sowie eine flache bzw. kompakte Bauweise aus. Ferner ist bei der Innendurchspülung mit einem Reinigungsfluid bei Zylinderrollenlagern und/oder Nadellagern gegenüber kugeligen Wälzkörpern bzw. Kugellagern die Neigung zu einem ungleichmäßigen bzw. pulsierenden Strömungsverhalten verringert, was durch die verringerte Spaltkanalbreite zwischen der äußeren Mantelfläche des Lagerkäfigs und der inneren Umfangsfläche des Schaftabschnitts bewirkt wird. Auch ist aufgrunddessen die Reinigungswirkung vorteilhaft erhöht.

Vorzugsweise weisen das zumindest eine distale Wälzlager und/oder das zumindest eine proximale Wälzlager, vorzugsweise alle Wälzlager der inneren Wälzlagerung, keramische Wälzkörper, bspw. keramische Nadeln, auf. Dadurch ist die mechanische Belastbarkeit, insb. das Dauerbelastungsverhalten, verbessert, was positiv zu längeren Standzeiten bzw. Wartungsintervallen führt. Weiterhin können die Wälzkörper noch geringer dimensioniert werden, so dass eine noch flacher ausgeführte Wälzkörperkonstruktion umsetzbar ist. Dadurch kann die Spaltkanalbreite zwischen der äußeren Mantelfläche des Lagerkäfigs und der inneren Umfangsfläche des Schaftabschnitts noch weiter verringert werden. Es ist auch denkbar, vollkeramische Lager zu verwenden. Insbesondere bestehen zusätzlich zu den Wälzkörpern auch die Lagerringe aus keramischen Werkstoffen.

Als ein zweiter Aspekt der vorliegenden Offenbarung wird ein chirurgisches Instrument mit einem offenbarungsgemäßen Instrumentenhandstück und einem, vorzugsweise drehbar angetriebenen und/oder antreibbaren, Werkzeug vorgeschlagen. Vorzugsweise umfasst ein Werkzeug einen Fräser, bswp. einen feinen oder groben Diamantfräser, einen ("Twin-Cut") Kugelfräser, einen Pinfräser, einen spiraligen oder geraden Kraniotomfräser, usw. und/oder einen Bohrer, bspw. einen Spiralbohrer, und/oder einen Polierkopf und/oder ein Drehmesser. Das Werkzeug kann außerdem ein nicht bewegtes Werkzeug sein, z.B. ein elektrisches Skalpell, ein Kauter, ein Laser oder dergleichen. Nominale Durchmesser des Werkzeugs können vorzugsweise von 1,0 mm bis 6,0 mm betragen.

Als ein dritter Aspekt der vorliegenden Offenbarung wird ein medizinisches Produktset mit zumindest einem ersten offenbarungsgemäßen Instrumentenhandstück in Kombination mit zumindest einem Zubehörteil vorgeschlagen. Vorzugsweise handelt es sich bei dem medizinischen Produktset um eine anwendungsbezogene Zusammenstellung für einen Operateur wie einen Chirurgen. Zunächst ist es bevorzugt, dass ein offenbarungsgemäßes Instrumentenhandstück mit einem offenbarungsgemäßen chirurgischen Instrument kombiniert ist. Alternativ oder kumulativ umfasst die Kombination mit zumindest einem Zubehörteil des Produktsets eine Vielzahl unterschiedlicher, insbesondere drehbar angetriebener und/oder antreibbarer, medizinischer Werkzeuge. Dabei betrifft dies vorzugsweise eine Zusammenstellung der Werkzeuge von unterschiedlichen Funktionen bzw. Typen, wie bspw. Bohrer, Fräser, usw., und/oder von gerader und/oder gebogener Form und/oder nach unterschiedlichen Größen, wie bspw. pädiatrisch, standard bzw. kurz, lang, usw., und/oder nach Härtegraden und/oder nach Materialien. Alternativ oder kumulativ umfasst die Kombination mit zumindest einem Zubehörteil des Produktsets ein zweites offenbarungsgemäßes Instrumentenhandstück, wobei das erste Instrumentenhandstück und das zweite Instrumentenhandstück unterschiedliche erste Durchmesser und/oder unterschiedliche zweite Durchmesser und/oder unterschiedliche erste Längen und oder unterschiedliche zweite Längen aufweisen. Alternativ oder kumulativ umfasst die Kombination mit zumindest einem Zubehörteil einen Werkzeugschlüssel zum Einsetzen eines zugehörigen Werkzeuges in das Instrumentenhandstück.

Ein solches Produktset weist den besonderen Vorteil auf, dass herstellerseitig sichergestellt wird, dass die von einem Anwender wie dem Operateur und/oder einem Klinikpersonal verwendeten Zubehörteile optimal zueinander passend bzw. funktional aufeinander abgestimmt sind bzw. werden können. Ein solches Produktset wird von dem Anwender als besonders nützlich angesehen. Weitere anwendungstechnische Vorteile zeigen sich in einer erhöhten Flexibilität, einer sichereren Handhabung sowie verbesserten logistischen Klinikabläufen, sowohl in der OP-Vorbereitung als auch in der Zentralen Sterilgutversorgungsabteilung (ZSVA).

Als ein vierter Aspekt der vorliegenden Offenbarung wird eine Spülvorrichtung wie ein Reinigungs- und Desinfektionsgerät, welche zur Innendurchspülung eines offenbarungsgemäßen Instrumentenhandstücks eingerichtet ist, vorgeschlagen. Ein Reinigungs- und Desinfektionsgerät (RDG), bzw. Thermodesinfektor genannt, dient der maschinellen Aufbereitung von wiederverwendbaren Medizinprodukten wie chirurgischen Instrumenten. Somit wird der Zentralen Sterilgutversorgungsabteilung (ZSVA) eine herstellerseitig auf die Reinigung des offenbarungsgemäßen Instrumentenhandstücks optimal abgestimmte Vorrichtung bereitgestellt.

Als ein fünfter Aspekt der vorliegenden Offenbarung wird ein Reinigungsverfahren zur Innendurchspülung eines offenbarungsgemäßen Instrumentenhandstücks in Strömungsrichtung von proximal nach distal in einer offenbarungsgemäßen Spülvorrichtung vorgeschlagen. Auf diese Weise sind Reinigungseffizienz bzw. der erreichbare Sterilisationsgrad verbessert.

Dabei ist vorzugsweise das offenbarungsgemäße Reinigungsverfahren weiter hydrodynamisch optimiert und derart ausgebildet, dass Stromlinien eines Reinigungsfluides, welche vorzugsweise durch das proximale Wälzlager laufen, solche Stromlinien umfassen, welche entlang einer äußeren Mantelfläche des durchgehend ausgebildeten Lagerkäfigs und/oder durch das zumindest eine distale Wälzlager laufen. Dadurch kann eine nicht vollständige bzw. unzureichende Durchströmung mit Reinigungsfluid weiter vermieden werden.

Abschließend sei darauf hingewiesen, dass das offenbarungsgemäße Instrumentenhandstück nicht auf die Verwendung alleinig bei der Chirurgie beschränkt ist. Die Offenbarung ist gleichermaßen für ähnliche medizinische Verwendungen vorteilhaft einsetzbar, insbesondere für die Vielfalt von zahnmedizinischen wie orthopädischen Situationen bzw. Maßnahmen sowie von Diagnoseverfahren bzw. Untersuchungsmethoden. Das Einsatzfeld umfasst sowohl die Humanmedizin wie die Veterinärmedizin. Der Erfindungsgedanke richtet sich auf jegliches Anwendungsfeld eines Instrumentenhandstücks zur Aufnahme eines, insbesondere drehbar gelagerten, Werkzeugs, bei welcher eine zuverlässige Reinigung per Innendurchspülung nach Gebrauch bzw. Werkzeugentnahme von wesentlicher Bedeutung für eine Wiederverwendung und/oder bei welcher eine möglichst kleine distale Bauweise essentiell ist.

Der Schutzbereich der vorliegenden Offenbarung ist durch die Ansprüche gegeben und wird durch die in der Beschreibung erläuterten oder in den Figuren gezeigten Merkmale nicht beschränkt.

### Kurzbeschreibung der Figuren

Fig. 1 ist eine leicht perspektivische Seitenansicht eines Instrumentenhandstücks (ohne Werkzeug) in einer Ausführungsform nach dem Stand der Technik;
Fig. 2 ist eine Seitenansicht des Instrumentenhandstücks (ohne Werkzeug) in der Ausführungsform nach dem Stand der Technik;
Fig. 3a ist ein distaler Detailausschnitt einer seitlichen Schnittansicht des Instrumentenhandstücks (ohne Werkzeug) in der Ausführungsform nach dem Stand der Technik, insb. die innere Wälzlagerung für ein Werkzeug veranschaulichend;
Fig. 3b ist, in Entsprechung von dem distalen Detailausschnitt der Fig. 3a, eine schematische Darstellung von hydrodynamischen Stromlinien, insb. veranschaulichend die innere Durchströmung mit einem Reinigungsfluid;
Fig. 4 ist eine leicht perspektivische Seitenansicht des offenbarungsgemäßen Instrumentenhandstücks (ohne Werkzeug) in einer bevorzugten Ausführungsform;
Fig. 5 ist eine Seitenansicht des offenbarungsgemäßen Instrumentenhandstücks (ohne Werkzeug) in der bevorzugten Ausführungsform;
Fig. 6a ist ein distaler Detailausschnitt einer seitlichen Schnittansicht des offenbarungsgemäßen Instrumentenhandstücks (ohne Werkzeug) in der bevorzugten Ausführungsform, insb. die innere Wälzlagerung für ein Werkzeug veranschaulichend;
Fig. 6b ist, in Entsprechung von dem distalen Detailausschnitt der Fig. 6a, eine schematische Darstellung von hydrodynamischen Stromlinien, insb. veranschaulichend die innere Durchströmung des offenbarungsgemäßen Instrumentenhandstücks (ohne Werkzeug) mit einem Reinigungsfluid gemäß der bevorzugten Ausführungsform;
Fig. 7 ist eine leicht perspektivische Seitenansicht eines bevorzugten Lagerkäfigs in Form einer Auszugsdarstellung als separates Bauteil für das Innere des offenbarungsgemäßen Instrumentenhandstücks gemäß der bevorzugten Ausführungsform;
Fig. 8a ist eine erste Schnittansicht des Instrumentenhandstücks (ohne Werkzeug), einen zu einem proximalen Wälzlager proximalen Bereich einer Werkzeugaufnahme in einem Schaftabschnitt zeigend, in der Ausführungsform nach dem Stand der Technik;
Fig. 8b ist eine, zu der ersten Schnittansicht der Fig. 8a distale, zweite Schnittansicht des Instrumentenhandstücks (ohne Werkzeug), das proximale Wälzlager in dem Schaftabschnitt zeigend, in der Ausführungsform nach dem Stand der Technik;
Fig. 9 ist eine Schnittansicht des offenbarungsgemäßen Instrumentenhandstücks, einen zu dem Schaftabschnitt proximalen Bereich im Übergang zu einem Griffabschnitt zeigend;
Fig. 10a ist eine erste Schnittansicht des offenbarungsgemäßen Instrumentenhandstücks (ohne Werkzeug), den zu dem proximalen Wälzlager proximalen Bereich der Werkzeugaufnahme im Schaftabschnitt zeigend, gemäß der bevorzugten Ausführungsform; und
Fig. 10b ist eine, zu der ersten Schnittansicht der Fig. 10a distale, zweite Schnittansicht des offenbarungsgemäßen Instrumentenhandstücks (ohne Werkzeug), einen mittigen Bereich des Lagerkäfigs gemäß Fig. 7 im distalen verengten Spitzenabschnitt zeigend, gemäß der bevorzugten Ausführungsform.

### Beschreibung des Ausführungsbeispiels

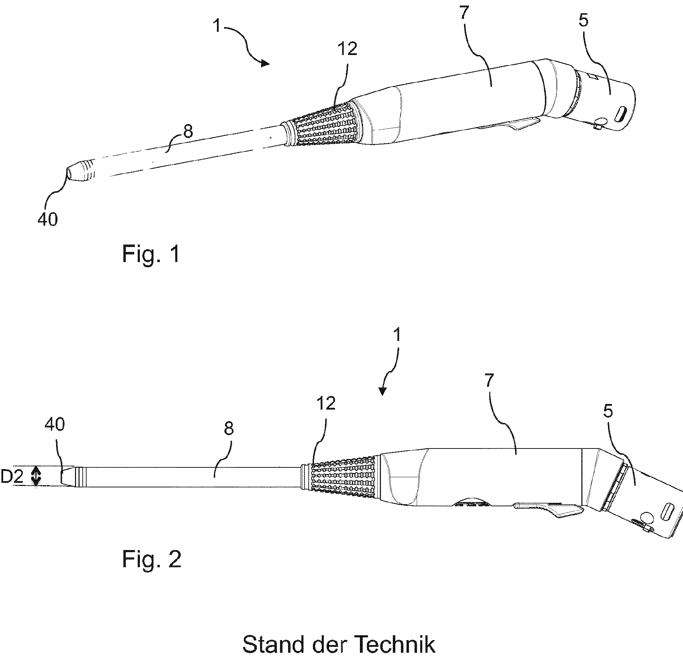

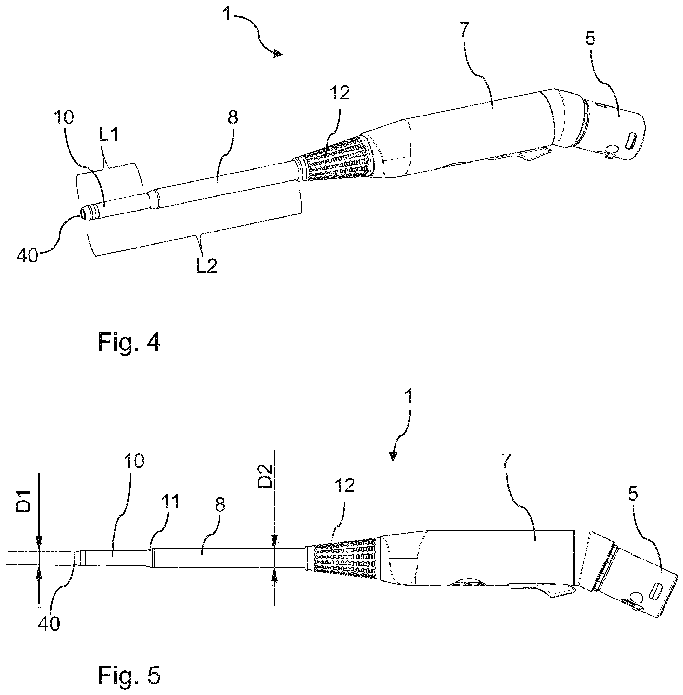

Nachstehend wird ein Ausführungsbeispiel der vorliegenden Offenbarung auf der Basis der zugehörigen Figuren 4 bis 6b, Fig. 7, sowie Figuren 9 bis 10bbeschrieben und insofern einem Ausführungsbeispiel nach dem Stand der Technik gemäß den Figuren 1 bis 3b sowie Figuren 8a und 8b gegenübergestellt. Daraus ergeben sich weitere Einzelheiten, Merkmale und Vorteile der Offenbarung.

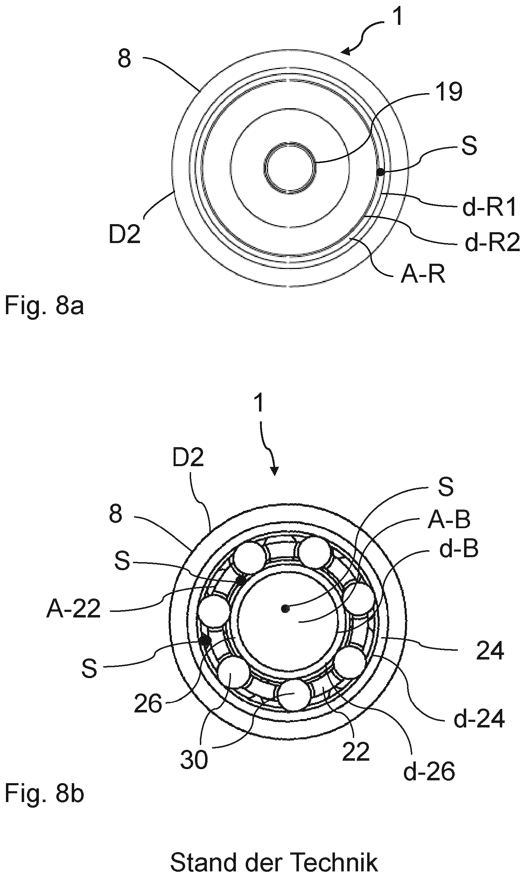

Insofern als das offenbarungsgemäße Instrumentenhandstück nach der bevorzugten Ausführungsform gemäß den Figuren 4 bis 6b sowie den Figuren 10a und 10b dem Ausführungsbeispiel nach dem Stand der Technik gemäß den analogen Figuren 1 bis 3b sowie den analogen Figuren 8a und 8b gleicht bzw. nicht ein offenbarungsgemäßes Unterschiedsmerkmal diskutiert wird, wird zur Vermeidung von Wiederholungen auf die einleitende Beschreibung bzw. die Bezeichnungen zum Stand der Technik verwiesen.

Fig. 1 bzw. Fig. 2 zeigen eine leicht perspektivische Seitenansicht bzw. eine Seitenansicht eines Instrumentenhandstücks (ohne Werkzeug) in einer Ausführungsform nach dem Stand der Technik. Ein chirurgisches Instrumentenhandstück 1 für ein chirurgisches Instrument umfasst einen integral ausgebildeten Griffabschnitt 7, welcher proximal bzw. Patienten-abgewandt durch einen (nicht abgebildeten) Operateur gehandhabt werden kann, sowie einen Schaftabschnitt 8, der sich in distaler bzw. Patienten-zugewandter Längsrichtung vom Griffabschnitt 7 erstreckt. Dabei ist ein Werkzeug (nicht abgebildet), wie beispielsweise ein Diamantfräser oder ein Spiralbohrer, an dem dem Griffabschnitt 7 gegenüberliegenden, distalen Ende in einer Zylinderbohrung 40 als distaler Austrittsöffnung des Schaftabschnitts 8 durch einen Nutzer anordbar. Dabei umfasst das Werkzeug (nicht abgebildet) typischerweise einen Werkzeugkopf, wie einen Bohr-, Fräs-, Schleif- oder Polierkopf, und einen Werkzeugschaft zum Einsetzen in die Zylinderbohrung 40.

Ferner weist das Instrumentenhandstück 1 endseitig proximal (Patienten-abgewandt) einen Anschluss 5 auf, mittels dem es an einen Drehmoment-Übertragungszug, eine Antriebseinheit, eine Energieversorgungseinheit oder ähnliches, wie aus dem Stand der Technik bekannt, anzuschließen ist.

Zwischen der Zylinderbohrung 40 an dem, für die Aufnahme des Werkzeugs eingerichteten, distalen Ende und dem Anschluss 5 ist ein Griffabschnitt 7 mit einer Oberflächen-Profilierung 12 (Noppen, Riefen, etc.) ausgebildet, an den sich ein zylindrischer Schaftabschnitt 8 in Richtung nach distal anschließt. Die Oberflächen-Profilierung 12 besteht aus radialen und axialen Vertiefungen, zwischen denen Erhebungen ausgebildet sind. Üblicherweise wird das Instrumentenhandstück 1 vom Operateur am Griffabschnitt 7 ergriffen und während des Einsatzes gehandhabt.

Der zylindrische Schaftabschnitt 8 ist mit einem konstanten zweiten Durchmesser D2 (siehe Fig. 2) ausgebildet.

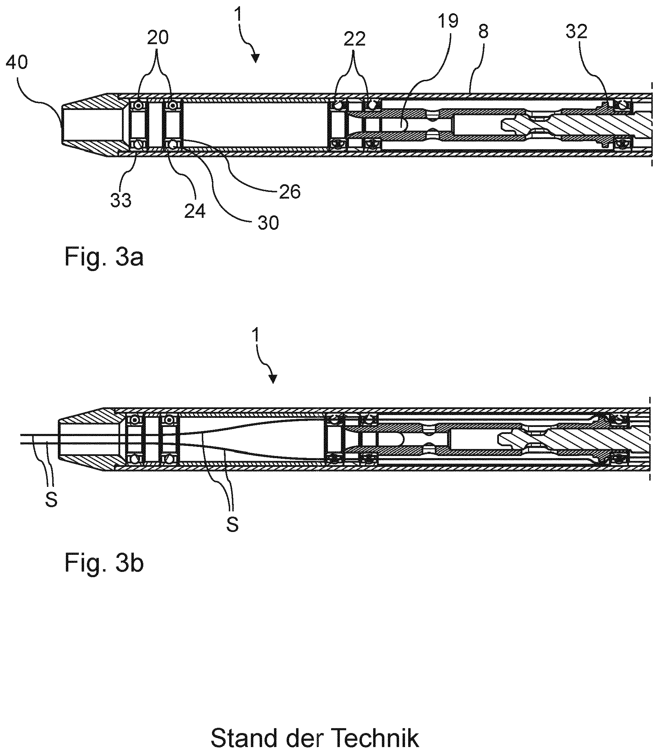

Ferner zeigen die Fig. 3a bzw. 3b jeweilig denselben distalen Detailausschnitt einer seitlichen Schnittansicht des Instrumentenhandstücks (ohne Werkzeug) nach dem Stand der Technik: zum einen (Fig. 3a) undurchströmt im Sinne einer herkömmlichen Werkstattzeichnung, zum anderen in Art einer hydrodynamischen Schemadarstellung in einem fluiddurchströmten Zustand (Fig. 3b). In Fig. 3b sind aus Gründen der Übersichtlichkeit nur die Stromlinien S mit einem Bezugszeichen versehen, weshalb im Sinne der nachfolgenden Beschreibung auf die Bezeichnung der Komponenten mit Bezugszeichen in der zugehörigen Fig. 3a verwiesen wird.

Diese Darstellungen der Fig. 3a bzw. 3b lassen insb. die innere gesamte Wälzlagerung erkennen. Diese ist zur drehbaren Anordnung eines rotatorisch antreibbaren Werkzeugs (nicht abgebildet) im Inneren des Instrumentenhandstücks 1, insb. des Schaftabschnitts 8, vorgesehen. Insofern sind die Ansichten der Fig. 3a bzw. 3b unterbrochen, wie durch die strichpunktierte Linie (am rechten Bildrand) angedeutet sein soll. Die gesamte Wälzlagerung umfasst ein distales Kugellagerpaar 20, gebildet aus zwei distalen Wälzlagern (links im Bild), und ein proximales Kugellagerpaar 22, gebildet aus zwei proximalen Wälzlagern 22 (rechts im Bild). Dabei sind alle vier einzelnen Kugellager, nämlich die jeweiligen des distalen Kugellagerpaars 20 und des proximalen Kugellagerpaars 22, jeweils gleich aufgebaut. Dazu umfasst jedes einzelne Kugellager eine Vielzahl Kugeln 30 als runde Wälzkörper, welche zwischen einem jeweiligen Innenring 26 und einem jeweiligen Außenring 24 rollen bzw. sich auf diesen, diese zueinander beabstandend, abwälzen. Der jeweilige Außenring 24 ist in einen distalen zylindrischen Innenflächenabschnitt 33 des Schaftabschnittes 8 eingepasst.

Ferner sind in dieser Schnittansicht der Fig. 3a bzw. 3b am proximalen Ende des Schaftabschnitts 8 eine innere Werkzeugaufnahme 19 zur Halterung bzw. Verankerung des Werkzeugsschafts (nicht abgebildet) sowie eine Führungshülse 32 erkennbar. Das durch die Zylinderbohrung 40 am distalen Werkzeugende eingeführte Werkzeug (nicht abgebildet) ist in der Werkzeugaufnahme 19 und Führungshülse 32 des Instrumentenhandstücks 1 vorzugsweise auswechselbar gehalten bzw. gekuppelt und kann über den proximalen Anschluss 5 (siehe Figuren 1 und 2) rotatorisch angetrieben werden.

Die hydrodynamische Schemadarstellung der Fig. 3b veranschaulicht einen fluiddurchströmten Zustand des Instrumentenhandstücks 1 anhand von linienförmig eingezeichneten Stromlinien S. Ein solcher fluiddurchströmter Zustand entsteht, wenn das Instrumentenhandstück 1 in eine (nicht abgebildete) Spülvorrichtung wie in ein Reinigungs- und Desinfektionsgerät eingesetzt wird. Die länglichen Stromlinien S repräsentieren eine Innendurchspülung des Instrumentenhandstücks 1 mit einem Reinigungsfluid, vorzugsweise mit einer hydrophilen oder lipophilen Reinigungslösung, in Strömungsrichtung von proximal nach distal. Insofern treten die Stromlinien S aus der distalen Austrittsöffnung 40 (links im Bild) aus.

Was die Durchströmung der gesamten Wälzlagerung in Richtung von dem proximalen Kugellagerpaar 22 (rechts im Bild) hin zu dem distalen Kugellagerpaar 20 (links im Bild) betrifft, so wird diese anhand des Verlaufs der Stromlinien S der Fig. 3b gut erkennbar: Zunächst findet eine, durch den konstruktiven Aufbau in proximaler Richtung bedingte, Zwangsdurchströmung - und damit hydrodynamisch wirksame Reinigung - des proximalen Kugellagerpaars 22 unter Umströmung der zugehörigen Kugeln 30 statt. Beim Austritt aus dem proximalen Kugellagerpaar 22 sucht sich jedoch die Strömung stromabwärts bzw. nach distal einen Strömungsweg im Wesentlichen zur Mittelachse des Schaftabschnitts hin (entsprechend dem Weg des geringsten Strömungswiderstands). Zuletzt strömt der Großteil des Reinigungsfluides durch die vergleichsweise große zylindrische Öffnung bzw. Bohrung der paarigen Innenringe 26, 26 des distalen Kugellagerpaares 20 und tritt sodann via der distalen Austrittsöffnung 40 aus dem Inneren des Schaftabschnitts 8 aus. Insofern wird das distale Kugellagerpaar 20 (links im Bild) nach dem Stand der Technik kaum durchströmt und somit auch nicht hinreichend (fluid)gereinigt.

Der vorstehend beschriebene Verlauf der Durchströmung bzw. die Strömungsverhältnisse, von proximal nach distal, durch das Instrumentenhandstück 1 in der Ausführungsform nach dem Stand der Technik wird weiter im Detail noch anhand der Figuren 8a und 8b veranschaulicht. So zeigt Fig. 8a eine erste Schnittansicht des herkömmlichen Instrumentenhandstücks 1 (ohne Werkzeug), welche einen Querschnitt durch den zylindrischen Schaftabschnitt 8 mit einem konstanten zweiten Durchmesser D2 (siehe auch Fig. 2) betrifft. Dabei liegt die erste Schnittansicht der Fig. 8a in einem Bereich der Werkzeugaufnahme 19 (siehe auch Fig. 3a), welcher proximaler als das proximale Wälzlager 22 (Fig. 22) angeordnet ist. Ferner zeigt Fig. 8b eine zweite Schnittansicht des herkömmlichen Instrumentenhandstücks (ohne Werkzeug), welche zu der ersten Schnittansicht der Fig. 8a distal bzw. in Strömungsrichtung weiter stromab geschnitten ist. Dabei schneidet die zweite Schnittansicht der Fig. 8b das proximale Wälzlager 22 in dem Schaftabschnitt 8 mit dem zweiten Durchmesser 8.

Dabei wird bei der ersten und zweiten Schnittansicht (für den Stand der Technik: in den Figuren 8a und 8b) insbesondere auf die hydrodynamische Schemadarstellung (für den Stand der Technik: in der Fig. 3b) Bezug genommen, welche den fluiddurchströmten Zustand des Instrumentenhandstücks 1 anhand der linienförmig eingezeichneten Stromlinien S (bzw. einer exemplarischen Auswahl der zwei Stromlinien aus real einer Vielzahl) veranschaulicht.

Dabei treten die Stromlinien S aus einer auf die Darstellung der Schnittansicht(en) (für den Stand der Technik: in den Figuren 8a und 8b) bezogenen Blattebene heraus; sprich (ideell) punktförmig in Richtung des Betrachters. Wie, sozusagen, eine Pfeilspitze eines dementsprechend zugehörigen Strömungsvektors einer Durchflussgeschwindigkeit, welcher durch die Blattebene durchtritt, ist eine jeweilige (exemplarisch bzw. in Auswahl dargestellte) Stromlinie S punktförmig dargestellt.

Damit bezeichnet die jeweilige, in der Schnittansicht punktförmige Stromlinie S eine jeweilig durchströmte bzw. offene Querschnittsfläche bzw. eine (zugehörige) Strömungsquerschnittsfläche. Mit anderen Worten, deutet ein mit der Stromlinie S in einer der Schnittansichten bezeichneter Punkt jeweilig eine (zwischen Konturlinien bzw. Körperkanten erkennbare bzw. einzelne) (Strömungs-)Querschnittsfläche an, welche jeweilig einem Strömungsweg der Durchströmung zur Verfügung steht. Somit steht zur bzw. bei der Durchspülung mit dem Reinigungsfluid mittels der (nicht abgebildeten) Spülvorrichtung, vorzugsweise mittels dem Reinigungs- und Desinfektionsgerät, die (jeweilige Strömungs-)Querschnittsfläche mit einem Eingangsanschluss des Instrumentenhandstücks 1 in Fluidverbindung.

Der ersten Schnittansicht der Figur 8a ist zu entnehmen, dass eine zylindrische Ringspalt-Querschnittsfläche A-R zwischen einem äußeren zylindrischen Ringspalt-Außendurchmesser d-R1 und einem inneren Ringspalt-Innendurchmesser d-R2, zur Durchströmung (mit punktförmig austretender Stromlinie S), ausgebildet ist. Somit berechnet sich die Ringspalt-Querschnittsfläche A-R als eine (jeweilige Strömungs-) Querschnittsfläche aus der Subtraktion der beiden Kreisflächen mit dem Ringspalt-Außendurchmesser d-R1 bzw. mit dem Ringspalt-Innendurchmesser d-R2 jeweils. Bspw. kann die Ringspalt-Querschnittsfläche A-R, wie in Fig. 8a gezeigt, 2,4 mm² betragen.

Die zweite Schnittansicht der Figur 8b zeigt die Strömungssituation stromab bzw. distal. Hier in dem proximalen Wälzlager 22 durchströmt die Durchströmung zwei durch den Innenring 26 getrennte Strömungsräume bzw. jeweilige Strömungs-) Querschnittsflächen. Dabei ergeben diese zusammen einen Gesamt-Wälzlager-Strömungsquerschnitt (bspw. 8,5 mm²), wie im Folgenden erläutert: Das proximale Wälzlager 22 ist mit sieben Kugeln 30 als den Wälzkörpern dargestellt, welche zwischen dem Innenring 26 mit dem Innenring-Durchmesser d-26 und dem, in den Schaftabschnitt 8 eingepassten, Außenring 24 mit dem Außenring-Durchmesser d-26 abwälzend rollen. Ein Teil der Durchströmung fließt (mit einer punktförmig austretenden Stromlinie S) mittig durch einen zylindrischen Bohrungs-Innenraum des Innenrings 26 mit einer Bohrungs-Querschnittsfläche A-B (bspw. 4,5 mm²) gemäß einem Bohrungs-Durchmesser d-B (bspw. 2,4 mm).

Zusätzlich wird von dem anderen Teil der Durchströmung ein zwischen dem Innenring 26 und dem Außenring 24 ausgebildeter und von den (sieben) Kugeln 30 freier Wälzlager-Innenraum des proximalen Wälzlagers 22 mit einer (freien) Wälzlager-Querschnittsfläche A-22 (bspw. 4,0 mm²), mit zwei punktförmig austretenden Stromlinien S, durchströmt.

Gemäß dem Strömungsgesetz des Weges gemäß dem geringsten Strömungswiderstand und/oder gemäß der als Wandhaftungsbedingung bekannten Strömungsbedingung, insb. im Falle einer Rohrströmung, und/oder gemäß der Durchströmung einer Schüttung ("Pre-Darcy") ergibt sich hydrodynamisch eine Verteilung der Durchflussgeschwindigkeit (in der Längsrichtung des Instrumentenhandstückes 1) mit einem Maximum innerhalb der Bohrungs-Querschnittsfläche A-B. Es stellt sich demgegenüber der andere Teil der Durchströmung durch die Wälzlager-Querschnittsfläche A-22 nur zu einem geringen bzw. gegenüber dem einen Teil vergleichsweise geringeren Anteil ein.

Mit anderen Worten bewirkt der Aufbau des herkömmlichen Instrumentenhandstückes 1 in unvorteilhafter Weise, dass bei dessen Durchströmung mit Reinigungsfluid gerade das Wälzlager (bzw. das hier stellvertretend hydrodynamisch diskutierte proximale Wälzlager 22) nur gering bzw. schwach bzw. langsam durchströmt wird. Als ganz besonders nachteilig ist der Umstand im Stand der Technik anzusehen, wobei sich der Strömungsquerschnitt von proximal (die Ringspalt-Querschnittsfläche A-R bspw. 2,4 mm²) nicht nur nicht verjüngt, sondern sich sogar nach distal (Gesamt-Wälzlager-Strömungsquerschnitt bspw. 8,5 mm²) erheblich aufweitet. Infolgedessen besteht das Risiko, dass sich eine mechanische Reinigungswirkung mittels des Reinigungsfluids nicht ausreichend einstellt. Insbesondere bemisst sich die mechanische Reinigungswirkung nach der kinetischen Energie des Reinigungsfluids als hydrodynamische Größe, in welche wiederum die Durchflussgeschwindigkeit zum Quadrat eingeht.

Der vorstehend ausgeführte Nachteil der demzufolge in dem Wälzlager reduzierten Durchströmung und damit herabgesetzten Reinigungswirkung wiegt hinsichtlich des technischen Ziels von Hygiene, insbesondere einer vollständig zuverlässigen Sterilisation, umso schwerer, insofern gerade die großen Oberflächen der Wälzlager besonders viel Fläche zur Anhaftung von Verunreinigungen wie Keimen, Biofilmen und dergleichen bieten.

Anhand der ersten und zweiten Schnittansicht für das herkömmliche Instrumentenhandstück in den Figuren 8a und 8b geht somit der oben anhand der Fig. 3b bereits beschriebene Nachteil aus dem Stand der Technik besonders deutlich hervor, was nämlich die unzureichende (fluid)reinigende Wirkung der anhand der Stromlinien S visualisierten Durchströmung betrifft.

Nach der vorliegenden Offenbarung wird hier Abhilfe geschaffen. Figuren 4 bis 7 zeigen unterschiedliche Ansichten gemäß einem Ausführungsbeispiel eines offenbarungsgemäßen Instrumentenhandstücks 1. So zeigen Fig. 4 bzw. Fig. 5 [in Analogie zu Fig. 1 bzw. Fig. 2 für den Stand der Technik] eine leicht perspektivische Seitenansicht bzw. eine Seitenansicht des offenbarungsgemäßen Instrumentenhandstücks (ohne Werkzeug) in einer bevorzugten Ausführungsform.

Anders als beim Stand der Technik weist der Schaftabschnitt 8 im Bereich der distalen Austrittsöffnung 40 (links in Figuren 4 bis 6b) einen verengten distalen Spitzenabschnitt 10 auf. Der distale Spitzenabschnitt 10 ist vom einem zweiten Durchmesser D2 des Schaftabschnittes 8 hinunter auf einen kleineren ersten Durchmesser D1 verengt (siehe Fig. 5). Mit anderen Worten, unterscheidet sich die in den Figuren 4 bis 7 gezeigte bevorzugte Ausführungsform eines offenbarungsgemäßen Instrumentenhandstückes von dem herkömmlichen Instrumentenhandstück nach dem Stand der Technik, wie in den Figuren 1 bis 3b gezeigt, dadurch, dass der längliche Schaftabschnitt 8 mit einem zweiten Durchmesser D2 in seinem distalen Bereich der Zylinderbohrung 40 als distaler Austrittsöffnung einen verengten distalen Spitzenabschnitt 10 aufweist.

Wie in Fig. 4 mittels geschweiften Klammern bezeichnet, nimmt die erste Länge L1 des distalen Spitzenabschnitt 10 einen distalen Teilabschnitt der (gesamten) zweiten Länge L2 des (gesamten) Schaftabschnitts 8 ein. Zwischen dem distalen Spitzenabschnitt 10 mit dem ersten Durchmesser D1 und dem Schaftabschnitt 8 mit dem zweiten Durchmesser D2 ist umlaufend ein Absatz bzw. eine schräge Anstufung als Übergangsbereich 11 (siehe Fig. 5) gradiell auslaufend bzw. angeschrägt ausgeformt.

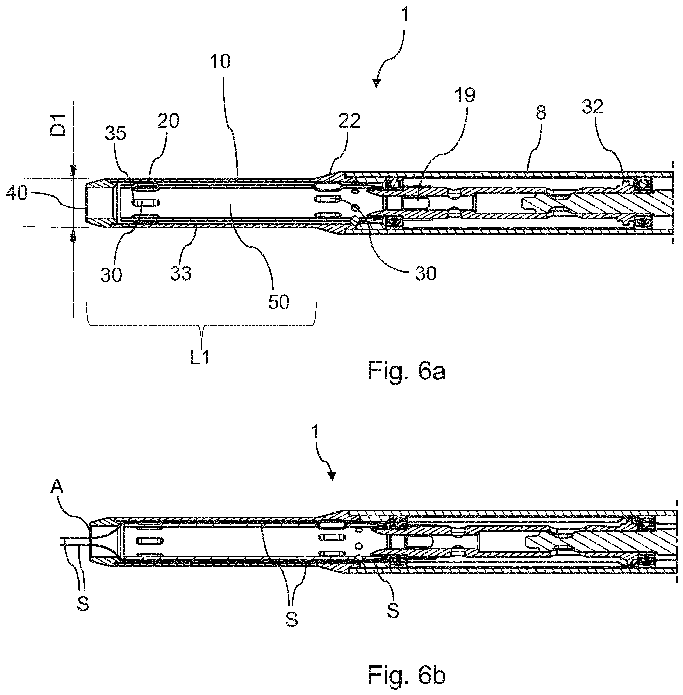

Ferner zeigen die Fig. 6a bzw. 6b [in Analogie zu Fig. 3a bzw. Fig. 3b für den Stand der Technik] jeweilig denselben distalen Detailausschnitt einer seitlichen Schnittansicht des offenbarungsgemäßen Instrumentenhandstücks (ohne Werkzeug) in der bevorzugten Ausführungsform: zum einen (Fig. 6a) undurchströmt im Sinne einer Werkstattzeichnung; zum anderen fluiddurchströmt (Fig. 6b). In Fig. 6b sind aus Gründen der Übersichtlichkeit nur die Stromlinien S sowie die Austritts-Querschnittsfläche A für die Strömung mit einem Bezugszeichen versehen, weshalb im Sinne der nachfolgenden Beschreibung auf die Bezeichnung der Komponenten mit Bezugszeichen in der zugehörigen Fig. 6a verwiesen wird.

Diese Darstellungen der Fig. 6a bzw. 6b lassen insb. die innere gesamte Wälzlagerung erkennen. Diese ist zur drehbaren Anordnung eines rotatorisch antreibbaren Werkzeugs (nicht abgebildet) ist im Inneren des Instrumentenhandstücks 1, insb. im distalen Spitzenabschnitt 10 des Schaftabschnitts 8, vorgesehen. Angedeutet durch die strichpunktierte Linie (am rechten Bildrand) sind die Ansichten der Fig. 6a bzw. 6b (wie bereits die der der Fig. 3a bzw. 3b) unterbrochen. Die gesamte Wälzlagerung umfasst ein distales Nadellager (bzw. Zylinderrollenlager) 20 (links im Bild) und ein baugleiches proximales Nadellager (bzw. Zylinderrollenlager) 22 (rechts im Bild). Dazu umfassen das distale Nadellager 20 und das proximale Nadellager 22 jeweils eine Vielzahl von, vorzugsweise keramischen, Nadeln 30 als elongierten bzw. länglichen unkugeligen Wälzkörpern, in gleichmäßigen Winkelsegmenten auf einem jeweiligen Kreisumfang verteilt. Die jeweiligen Nadeln 30 sind in einer Vielzahl zugehöriger Längsnuten 35 im Lagerkäfig 50 um ihre Längsmittenachse drehbeweglich eingefasst bzw. angeordnet. Das distale Nadellager 20 und das proximale Nadellager 22 sind mittels eines in die zylindrische Innenbohrung des distalen Schaftsabschnitts 10 eingesetzten zylindrischen Lagerkäfigs 50 zueinander, in Längsrichtung des Schaftabschnitts 8, beabstandet angeordnet. Insofern rollen bzw. wälzen die jeweiligen Nadeln 30 des distale Nadellagers 20 und des proximalen Nadellagers 22 innen auf einem zylindrischen Innenflächenabschnitt 33 des distalen Spitzenabschnitts 10 bzw. des Schaftsabschnitts 8 ab. Vergleichbar dem Stand der Technik sind am proximalen Ende des Schaftabschnitts 8 eine innere Werkzeugaufnahme 19 zur Halterung bzw. Verankerung des Werkzeugsschafts (nicht abgebildet) sowie eine Führungshülse 32 erkennbar.

Fig. 6b zeigt, wie bereits die Fig. 3b für den Stand der Technik, eine schematische Darstellung von hydrodynamischen Stromlinien S. Somit wird, sofern keine Unterschiede zum Stand der Technik betroffen sind, zur Vermeidung von Wiederholungen auf die Ausführungen zu Fig. 3b verwiesen. Im Gegensatz zu Fig. 3b veranschaulicht Fig. 6b einen offenbarungsgemäßen fluiddurchströmten Zustand bei innerer Durchströmung des offenbarungsgemäßen Instrumentenhandstücks (ohne Werkzeug) gemäß der bevorzugten Ausführungsform mit einem Reinigungsfluid. Ein solcher offenbarungsgemäßer fluiddurchströmter Zustand kann auf bevorzugte Weise dadurch bewirkt bzw. eingestellt werden, dass das offenbarungsgemäße Instrumentenhandstück 1 in eine (nicht abgebildete) offenbarungsgemäße Spülvorrichtung wie in ein Reinigungs- und Desinfektionsgerät eingesetzt wird. Die länglichen Stromlinien S repräsentieren eine Innendurchspülung des Instrumentenhandstücks 1 mit einem Reinigungsfluid, vorzugsweise mit einer hydrophilen oder lipophilen Reinigungslösung, in Strömungsrichtung von proximal nach distal (von rechts nach links im Bild). Insofern treten die Stromlinien S aus der distalen Austrittsöffnung 40 (links im Bild) des verengten distalen Spitzenabschnitts 10 mit einer entsprechend verringerten Austritts-Querschnittsfläche A der Strömung aus.

Anhand des Verlaufs der Stromlinien S der Fig. 6b ist die offenbarungsgemäße Durchströmung auch des distalen Nadellagers 20 als distalem Wälzlager gut erkennbar. Nahezu entlang der gesamten Länge des Schaftabschnitts 8, insbesondere auf der ersten Länge L1 des distalen Spitzenabschnitts, laufen die Stromlinien entlang des Innenflächenabschnitts 33 des distalen Spitzenabschnitts 10 bzw. des Schaftsabschnitts 8. Insbesondere sucht sich die Strömung erst nach der Passage auch des distalen Nadellagers 20 bzw. erst kurz vor dem Strömungsaustritt aus der distalen Austrittsöffnung 40 mit verringerter Austritts-Querschnittsfläche A einen Strömungsweg stromabwärts im Wesentlichen zur Mittelachse des Schaftabschnitts hin. Somit findet offenbarungsgemäß eine hydrodynamisch wirksame (Fluid-)Reinigung auch des distalen Nadellagers 20 als distalem Wälzlager statt.

Denn aufgrund des offenbarungsgemäß verengten distalen Spitzenabschnitts 10 sind zur Verbesserung der Reinigungswirkung die Strömungsgeschwindigkeit gemäß der verringerten Austritts-Querschnittsfläche A und der Druck in dem distalen Spitzenabschnitt 10 des Instrumentenhandstücks 1 erhöht. Dies bewirkt die Durchmesseränderung des distalen Spitzenabschnitts 10 vom Außendurchmesser 5,6 mm als beispielhaftem zweiten Durchmesser D2 zum Außendurchmesser 4,4 mm als beispielhaftem ersten Durchmesser D1 im Bereich der ersten 20 mm als beispielhafter erster Länge L1.

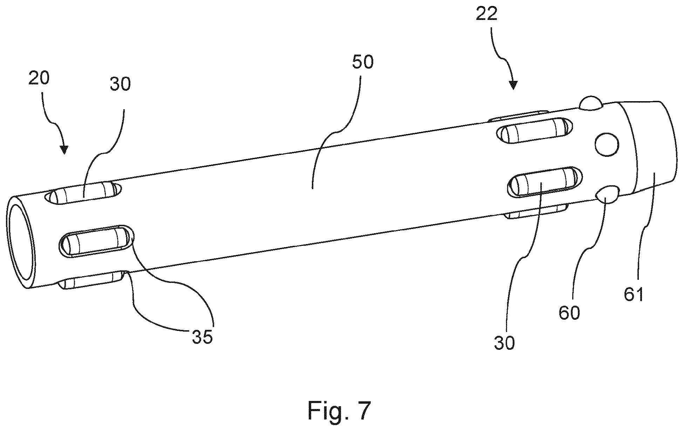

Darüberhinaus sorgt der spezielle konstruktive Aufbau des Lagerkäfigs 50 als durchgehendem Rohr für eine geringere Anschmutzung bei der Anwendung und gleichzeitig, durch die gezielte Führung des Reinigungsfluids, für eine optimierte Reinigung. Ein kleiner Anteil des Reinigungsfluides gelangt weiterhin durch die Werkzeugöffnung 40 als distaler Austrittsöffnung des Intrumentenhandstücks 1 und sorgt auch hier für eine optimale Reinigung, da keine störenden Teile den Fluss des Reinigungsfluids blockieren. Figuren 6a und 6b veranschaulichen die konstruktiven Details hinsichtlich des Einbaus bzw. der Montage des Lagerkäfigs 50, welcher in Fig. 7 als Einzelbauteil genauer zu sehen ist.

Der technische Effekt auf den Verlauf der Strömungslinien S aufgrund des konstruktiven Aufbaus sowie der Anordnung der besonders bevorzugten Ausführungsform mit einem Lagerkäfig 50 ist insbesondere wiederum anhand der Fig. 6b nachzuvollziehen. Es wird erkennbar, dass der Lagerkäfig 50 sogar eine Zwangsdurchströmung des distalen Nadellagers 20 bewirkt, sprich, dieses wird jedenfalls passiert und somit (fluid)gereinigt. Insbesondere wird durch den Einbau eines solchen Lagerkäfigs 50 zusätzlich vorteilhaft eine weitgehende Unabhängigkeit von einem proximal vorgelegten Eingangsdruck des Reinigungsfluids erreicht, was der Stabilität eines offenbarungsgemäßen Reinigungsverfahrens weiter zuträglich ist.

Fig. 7 zeigt, in Form einer vergrößernden Auszugsdarstellung unter Bezugnahme auf Fig. 6a bzw. Fig. 6b, eine leicht perspektivische Seitenansicht eines Lagerkäfigs 50 , so wie er als separates Bauteil einer Wälzlagerung für das Werkzeug im Inneren des offenbarungsgemäßen Instrumentenhandstücks 1 bevorzugt vorgesehen sein kann. Dieser Darstellung der Fig. 7 ist gut zu entnehmen, dass der Lagerkäfig 50 in Form eines zylindrischen Rohres ein distales Wälzlager 20 (links im Bild) und ein proximales Wälzlager 22 (rechts im Bild) beabstandet anordnet. Dabei sind das distale Wälzlager 20 und das proximale Wälzlager 22 mittels jeweilig fünf, am Umfang des Lagerkäfigs 50 gleichmäßig verteilten, Nadeln 30 als unkugeligen Wälzkörpern ausgeführt. Die Nadeln 30 ihrerseits sind in zugehörigen Längsnuten 35 des Lagerkäfigs 50 um ihre Längsachse drehbar bzw. wälzend angeordnet. Ferner sind am proximalen Ende des Lagerkäfigs 50 eine Vielzahl von runden Führungselementen 60 sowie eine Abgleitfläche 61 erkennbar.

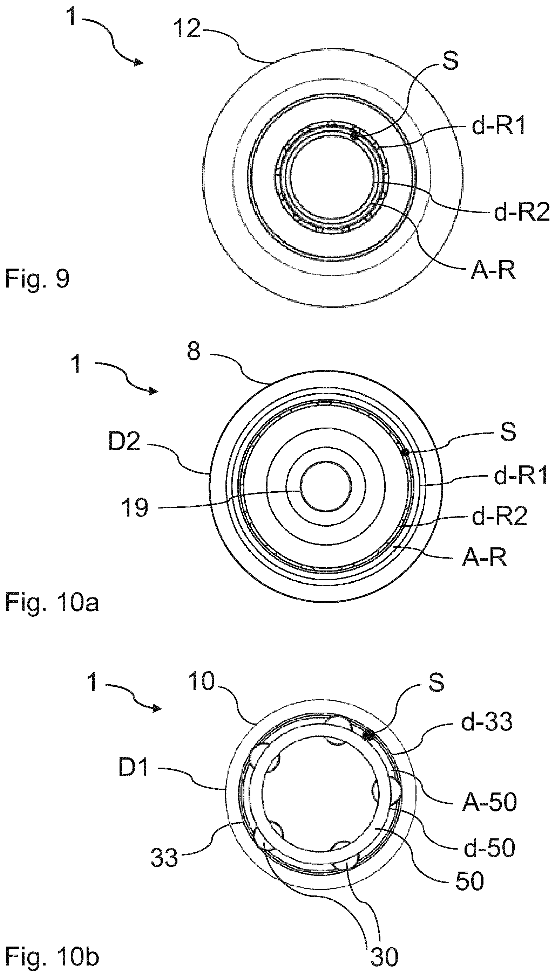

Fig. 9 zeigt eine Schnittansicht des offenbarungsgemäßen Instrumentenhandstücks 1, welche einen zu dem Schaftabschnitt 8 proximalen Bereich im Übergang 12 zu einem Griffabschnitt 7 (gemäß Figur 4) darstellt. Es ist zu entnehmen, dass eine dem Querschnitt der Fig. 9 zugehörige (zylindrische) Ringspalt-Querschnittsfläche A-R (bspw. ca. 3,6 mm²) zwischen einem zugehörigen äußeren zylindrischen Ringspalt-Außendurchmesser d-R1 (bspw. ca. 3,2 mm) und einem zugehörigen inneren Ringspalt-Innendurchmesser d-R2 (bspw. ca. 2,4 mm), zur Durchströmung (mit punktförmig austretender Stromlinie S), ausgebildet ist.

Ferner zeigen Fig. 10a bzw. Fig. 10b [in Analogie zu Fig. 8a bzw. Fig. 8b für den Stand der Technik] eine erste und eine zweite Seitenansicht des offenbarungsgemäßen Instrumentenhandstücks 1 (ohne Werkzeug) in der bevorzugten Ausführungsform gemäß der Figuren 4 bis 7: So zeigt zunächst Fig. 10a eine erste Schnittansicht in einem zu dem proximalen Wälzlager 22 (gemäß Figur 6a) proximalen Bereich der Werkzeugaufnahme 19 im Schaftabschnitt 8 mit dem zweiten Durchmesser D2.

Und weiter zeigt Fig. 10b eine, zu der ersten Schnittansicht der Fig. 10a distale, zweite Schnittansicht eines mittigen Bereichs des Lagerkäfigs 50 (gemäß Figuren 6a und 7) im offenbarungsgemäßen distalen verengten Spitzenabschnitt 10 mit dem ersten Durchmesser D1.

Analog zu vorstehender Diskussion für den Stand der Technik (anhand der diesbezüglichen Figuren 8a und 8b) wird bei der ersten und zweiten Schnittansicht der Figuren 10a bzw. 10b insbesondere auf die hydrodynamische Schemadarstellung in der Fig. 6b Bezug genommen, welche den fluiddurchströmten Zustand des Instrumentenhandstücks 1 anhand der linienförmig eingezeichneten Stromlinien S (bzw. einer exemplarischen Auswahl der zwei Stromlinien aus real einer Vielzahl) veranschaulicht.

Fig. 10a ist zu entnehmen, dass eine dem Querschnitt des Schaftabschnitts 8 in Fig. 10a zugehörige (zylindrische) Ringspalt-Querschnittsfläche A-R (bspw. ca. 3,6 mm²) zwischen einem zugehörigen äußeren zylindrischen Ringspalt-Außendurchmesser d-R1 (bspw. ca. 4,5 mm) und einem zugehörigen inneren Ringspalt-Innendurchmesser d-R2 (bspw. ca. 4,0 mm), zur Durchströmung (mit punktförmig austretender Stromlinie S), ausgebildet ist.

Es kann demnach insbesondere bevorzugt sein, dass die dem Querschnitt des Schaftabschnitts 8 in Fig. 10a zugehörige Ringspalt-Querschnittsfläche A-R der dem Querschnitt der Fig. 9 zugehörige (zylindrische) Ringspalt-Querschnittsfläche A-R in etwa entspricht (Flächenrelation zueinander von 90 % bis 110 %, weiter bevorzugt von 98 % bis 102 %, insb. ca 100 %). Dies bewirkt eine vorteilhafte Gleichmäßigkeit des Verlaufs des Spüldrucks bzw. der Durchflussgeschwindigkeit bzw. der kinetischen Energie in dem Instrumentenhandstück 1 von proximal nach distal.

Fig. 10b betreffs eines offenbarungsgemäßen distalen Querschnitts ist unter Bezugnahme auf Fig. 6a bzw. Fig. 6b zu entnehmen, dass der distale verengte Spitzenabschnitt 10 mit dem ersten Durchmesser D1 den zylindrischen (in Längsrichtung des Instrumentenhandstückes 1) durchgehenden Lagerkäfig 50 innen beabstandet umfasst. Zur Durchströmung (mit punktförmig austretender Stromlinie S) ist um den Lagerkäfig 50 herum ein zugehöriger Ringspalt ausgebildet. Dahingegen sind ist zumindest das proximale Ende, vorzugsweise auch das distale Ende, des Lagerkäfig 50 verschlossen, so dass das mittige Zylindervolumen des Lagerkäfigs 50 nicht durchströmt werden kann bzw. wird.

Hinter dem in der Fig. 10b gezeigten Querschnitt des distalen Spitzenabschnitts 10 durch einen (in Längsrichtung) mittigen Bereich des Lagerkäfigs 50 sind in Blickrichtung die fünf, am Umfang des Lagerkäfigs 50 gleichmäßig verteilten, Nadeln 30 als unkugelige Wälzkörper zu erkennen (vgl. Fig. 7).

Dabei ist der zugehörige Ringspalt innerhalb des Innenflächenabschnitts 33 an einer äußeren Mantelfläche des durchgehend ausgebildeten Lagerkäfigs 50 zwischen einem Innenflächenabschnitt-Durchmesser d-33 (bspw. ca. 3,8 mm) als einem Ringspalt-Außendurchmesser und einem Lagerkäfig-Außendurchmesser d-50 (bspw. ca. 3,3 mm) als einem Ringspalt-Innendurchmesser ausgebildet. Der um den Lagerkäfig 50 angeordnete Ringspalt weist dabei eine Lagerkäfig-Ringspalt-Querschnittsfläche A-50 (bspw. ca. 2,8 mm²) auf.

Vorzugsweise kann die Lagerkäfig-Ringspalt-Querschnittsfläche A-50 kleiner-gleich 3,5 mm², weiter bevorzugt kleiner-gleich ca. 3 mm² und insbesondere bevorzugt kleiner-gleich 2,8 mm² sein. Alternativ oder kumulativ kann die Lagerkäfig-Ringspalt-Querschnittsfläche A-50 kleiner-gleich einer Strömungsquerschnittsfläche sein, welche in einem dazu proximalen Bereich des Instrumentenhandstückes 1 frei durchströmbar ist, wie bspw. in den Figuren 10a und/oder 9 gezeigt. Weiter bevorzugt kann die Lagerkäfig-Ringspalt-Querschnittsfläche A-50 kleiner-gleich einer proximalen Schaftabschnitt-Ringspalt-Querschnittsfläche A-R (vgl. Fig. 9) sein.

Insbesondere wird offenbarungsgemäß der in Strömungsrichtung von proximal nach distal an der distalen Austrittsöffnung (mit Bezugszeichen 40, siehe Figuren 4 bis 6a) bzw. in der Lagerkäfig-Ringspalt-Querschnittsfläche A-50 anliegende bzw. vorliegende Spüldruck positiv erhöht. Damit bietet das offenbarungsgemäße Instrumentenhandstück Abhilfe gegenüber der im Stand der Technik technisch problematischen Situation, welche in Form eines ggf. zu niedrigen jeweiligen Spüldrucks insbesondere in einer Situation mit hoher klinischer Auslastung auftreten kann. Beispielsweise kann der in einem (nicht abgebildeten) Reinigungs- und Desinfektionsgerät (bspw. vom Typus "MIELE G 7825", Bau 80) als einer Spülvorrichtung von extern an eine Anzahl mehrerer angeschlossener und zu reinigender Instrumentenhandstücke vorgelegte gesamte proximale Eingangsdruck des Reinigungsfluides bei maximal ca. 1600 mbar im Sinne eines proximalen Absolutdruckes liegen. Daraus ergibt sich im Sinne einer Druckdifferenz bzw. eines Drucküberschüsses bzw. eines Druck-Deltas gegenüber dem atmosphärischen Umgebungsdruck von bspw. ca. 950 mbar ein maximaler (proximaler) Spüldruck für ein einzelnes angeschlossenes Instrumentenhandstück 1 von ca. 650 mbar. Dieser maximale (proximale) Spüldruck teilt sich jedoch bei Vorliegen bzw. Anschließen mehrerer Instrumentenhandstücke auf diese auf und ist damit auf einen jeweiligen Spüldruck verringert. Das beispielhafte Reinigungs- und Desinfektionsgerät stellt maximal 22 Anschlüsse ("Luer-Lock: Typ Miele"; Anschluss-Innendurchmesser 3 mm; somit Strömungsquerschnitt: A = ca. 7 mm²) bereit. Im Falle von 11 angeschlossenen Instrumentenhandstücken, also der Hälfte, konnte eine Reduktion des jeweiligen (proximalen) Spüldruckes auf ca. 500 mbar proximal festgestellt werden. Im Falle von 22 (von 22) angeschlossenen Instrumentenhandstücken konnte verzeichnet werden, dass sich der jeweilige (proximale) Spüldruck noch weiter auf ca. 315 mbar proximal verringerte bzw. abschwächte. Der jeweilige (proximale) Spüldruck steht also am Eingang des Instrumentenhandstücks zur Verfügung (proximaler Griffabschnitt-Strömungsquerschnitt bspw. Ø 1,7 mm, A = 2,27 mm²).

Der proximal von dem beispielhaften Reinigungs- und Desinfektionsgerät vorlegte (proximale) Spüldruck reduziert sich unter weiterer Berücksichtigung von diversen Strömungswiderständen wie der Rohrreibung entlang der inneren Durchströmung des Instrumentenhandstückes von proximal nach distal weiterhin auf einen distal vorliegenden (distalen) Spüldruck.

Die vorliegende Offenbarung gewährleistet, dass auch bei einem niedrigeren jeweiligen Spüldruck eine zuverlässige und hinreichende Fluidreinigung erfolgt. So bewirkt der verengte distale Spitzenabschnitt 10, dass auch noch im distalen Bereich ein wirksamer Spüldruck vorliegt. Insbesondere bewirkt die besonders bevorzugte Ausführungsform mit dem durchgehend ausgebildeten Lagerkäfig 50 eine kraftvolle Zwangsdurchströmung in der umgebenden Lagerkäfig-Ringspalt-Querschnittsfläche A-50. Somit erfolgt, trotz Strömungsdruckverlusten, noch stromab bzw. distal vom unverengten Schaftabschnitt 8, nämlich im distalen Spitzenabschnitt 10, eine wirksame Fluidreinigung. Diese stellt eine quasi unverminderte mechanische Reinigungsleistung in dem distalen Wälzlager 20 und in dem proximalen Wälzlager 22 sicher. Insofern erhält sogar das proximale Wälzlager 22 die volle Spülströmung des Reinigungsfluids.

### Bezugszeichen

- 1: Instrumentenhandstück
- 5: Anschluss
- 7: Griffabschnitt
- 8: Schaftabschnitt
- 10: distaler Spitzenabschnitt
- 11: Übergangsbereich
- 12: Profilierung
- 19: Werkzeugaufnahme
- 20: distales Wälzlager
- 22: proximales Wälzlager
- 24: Außenring
- 26: Innenring
- 30: Wälzkörper
- 32: Längsnut
- 33: Innenflächenabschnitt
- 35: Führungshülse
- 40: distale Austrittsöffnung
- 50: Lagerkäfig
- 60: Führungselement
- 61: Abgleitfläche
- A: Austritts-Querschnittsfläche
- A-B: Bohrungs-Querschnittsfläche
- A-R: Ringspalt-Querschnittsfläche
- A-22: Wälzlagerraum-Querschnittsfläche
- A-50: Lagerkäfig-Ringspalt-Querschnittsfläche
- D1: erster Durchmesser (des distalen Spitzenabschnitts)
- D2: zweiter Durchmesser (des Schaftabschnitts)
- d-B: Bohrungs-Durchmesser
- d-24: Außenring-Durchmesser (innen)
- d-26: Innenring-Durchmesser (außen)
- d-33: Innenflächenabschnitt-Durchmesser
- d-50: Lagerkäfig-Außendurchmesser
- d-R1: Ringspalt-Außendurchmesser
- d-R2: Ringspalt-Innendurchmesser
- L1: erste Länge (des distalen Spitzenabschnitts)
- L2: zweite Länge (des gesamten Schaftabschnitts)
- S: Stromlinie (Reinigungsfluid)

## Patentansprüche

1. Chirurgisches Instrumentenhandstück (1) für ein chirurgisches Instrument umfassend:
- einen Griffabschnitt (7) zur proximalen Handhabung durch einen Operateur, und
- einen Schaftabschnitt (8), der sich in distaler Längsrichtung vom Griffabschnitt (7) erstreckt, wobei der Schaftabschnitt (8) mit einer distalen Austrittsöffnung (40) an dem einen dem Griffabschnitt (7) gegenüberliegenden, distalen Ende des Schaftabschnitts (8) eingerichtet ist, um ein durch einen Nutzer auswechselbar anordenbares Werkzeug durch Einführen in die distale Austrittsöffnung (40) des Schaftabschnitts (8) in der inneren Werkzeugaufnahme (19) auswechselbar anzuordnen,
wobei der Schaftabschnitt (8) im Bereich der distalen Austrittsöffnung (40) zumindest einen, hinsichtlich einer Querschnittsfläche, verengten distalen Spitzenabschnitt (10) aufweist,
wobei der Schaftabschnitt (8) eine innere Wälzlagerung, die eingerichtet ist, das auswechselbar anordenbare Werkzeug drehbar zu lagern, vorzugsweise zumindest teilweise im Bereich des verengten distalen Spitzenabschnitts (10), umfasst,
wobei die Wälzlagerung zumindest ein distales Wälzlager (20) und zumindest ein proximales Wälzlager (22) umfasst und wobei das zumindest eine distale Wälzlager (20) und das zumindest eine proximale Wälzlager (22) durch einen zwischen ihnen durchgehend ausgebildeten Lagerkäfig (50) beabstandet angeordnet sind,
**dadurch gekennzeichnet, dass** der Schaftabschnitt (8) mit einer inneren Werkzeugaufnahme (19) an dem anderen, proximalen Ende des Schaftabschnitts eingerichtet ist.

2. Chirurgisches Instrumentenhandstück (1) nach Anspruch 1, wobei ein erster Durchmesser (D1) des verengten distalen Spitzenabschnitts (10):
- gegenüber einem zweiten Durchmesser (D2) eines unverengten Bereichs des Schaftabschnitts (8), um einen Durchmesser-Relationsfaktor von maximal 95 Prozent, vorzugsweise von maximal 85 Prozent, weiter bevorzugt von ca. 79 Prozent kleiner ist;
und/oder
- zwischen 3,5 und 5,3 Millimeter, vorzugsweise zwischen 4,0 und 5,0 Millimeter, weiter bevorzugt zwischen 4,3 und 4,5 Millimeter beträgt.

3. Chirurgisches Instrumentenhandstück (1) nach Anspruch 1 oder 2, wobei eine erste Länge (L1) des distalen Spitzenabschnitts (10):
- bezogen auf eine, gesamte, zweite Länge (L2) des Schaftabschnitts (8) einen Längenanteil von mindestens 5 Prozent, vorzugsweise von mindestens 20 Prozent, weiter bevorzugt von mindestens 35 Prozent bemisst; und/oder
- zwischen 5 und 40 Millimeter, vorzugsweise zwischen 10 und 30 Millimeter, weiter bevorzugt zwischen 18 und 22 Millimeter beträgt.

4. Chirurgisches Instrumentenhandstück (1) nach einem der vorhergehenden Ansprüche, wobei ein als Absatz von dem verengten distalen Spitzenabschnitt (10) zu dem unverengten Bereich des Schaftabschnitts (8) ausgebildeter Übergangsbereich (11) gerundet und/oder gradiell auslaufend und/oder angeschrägt geformt ist.

5. Chirurgisches Instrumentenhandstück (1) nach einem der vorhergehenden Ansprüche, wobei der Lagerkäfig (50) vollständig geschlossen ist oder zu einem geringen flächenbezogenen Lochvolumenanteil fluiddurchlässig ist, wobei vorzugsweise der Lochvolumenanteil kleiner 40 Prozent, weiter bevorzugt kleiner 15 Prozent, insbesondere bevorzugt kleiner 8 Prozent beträgt.

6. Chirurgisches Instrumentenhandstück (1) nach einem der vorhergehenden Ansprüche, wobei das zumindest eine distale Wälzlager (20) und/oder das zumindest eine proximale Wälzlager (22), vorzugsweise alle Wälzlager der inneren Wälzlagerung, nicht-kugelige Wälzkörper aufweisen, vorzugsweise als Zylinderrollenlager und/oder als Nadellager ausgebildet sind.

7. Chirurgisches Instrumentenhandstück (1) nach einem der vorhergehenden Ansprüche, wobei das zumindest eine distale Wälzlager (20) und/oder das zumindest eine proximale Wälzlager (22), vorzugsweise alle Wälzlager der inneren Wälzlagerung, keramische Wälzkörper aufweisen.

8. Chirurgisches Instrumentenhandstück (1) nach einem der vorhergehenden Ansprüche, wobei eine in dem distalen verengten Spitzenabschnitt (10) angeordnete Lagerkäfig-Ringspalt-Querschnittsfläche (A-50) eines Ringspaltes, welcher an einer äußeren Mantelfläche des durchgehend ausgebildeten Lagerkäfigs (50) zwischen einem Innenflächenabschnitt-Durchmesser (d-33) des Innenflächenabschnitts (33) als einem Ringspalt-Außendurchmesser und einem Lagerkäfig-Außendurchmesser (d-50) als einem Ringspalt-Innendurchmesser ausgebildet ist:
- kleiner-gleich 3,5 mm², weiter bevorzugt kleiner-gleich ca. 3 mm² und insbesondere bevorzugt kleiner-gleich 2,8 mm² beträgt; und/oder
- kleiner-gleich einer Strömungsquerschnittsfläche, welche in einem dazu proximalen Bereich des Instrumentenhandstückes (1) frei durchströmbar ist, ist, weiter bevorzugt kleiner-gleich einer proximalen Schaftabschnitt-Ringspalt-Querschnittsfläche (A-R) des Schaftabschnitts (8) ist.

9. Chirurgisches Instrument mit einem Instrumentenhandstück (1) nach einem der vorhergehenden Ansprüche und einem, vorzugsweise drehbar angetriebenen und/oder antreibbaren, Werkzeug.

10. Medizinisches Produktset, vorzugsweise eine anwendungsbezogene Zusammenstellung für einen Operateur wie einen Chirurgen, mit zumindest einem ersten Instrumentenhandstück (1) nach einem der vorhergehenden, auf das Instrumentenhandstück (1) gerichteten, Ansprüche, in Kombination mit zumindest:
- einem chirurgischen Instrument nach dem direkt voranstehenden Anspruch; und/oder
- mit einer Vielzahl unterschiedlicher, insbesondere drehbar angetriebener und/oder antreibbarer, medizinischer Werkzeuge, vorzugsweise in einer Zusammenstellung der Werkzeuge von unterschiedlichen Funktionen und/oder von gerader und/oder gebogener Form und/oder nach unterschiedlichen Größen und/oder Härtegraden und/oder Materialien; und/oder
- einem zweiten Instrumentenhandstück (1) nach einem der vorhergehenden, auf das Instrumentenhandstück (1) gerichteten, Ansprüche, wobei das erste Instrumentenhandstück (1) und das zweite Instrumentenhandstück (2) unterschiedliche erste Durchmesser (D1) und/oder unterschiedliche zweite Durchmesser (D2) und/oder unterschiedliche erste Längen (L1) und oder unterschiedliche zweite Längen (D2) aufweisen; und/oder
- einem Werkzeugschlüssel zum Einsetzen eines zugehörigen Werkzeuges in das Instrumentenhandstück (1).

11. Reinigungsverfahren zur Innendurchspülung eines erfindungsgemäßen Instrumentenhandstücks (1) nach einem der vorhergehenden, auf das Instrumentenhandstück (1) gerichteten, Ansprüche in Strömungsrichtung von proximal nach distal in einer Spülvorrichtung.

12. Reinigungsverfahren nach dem direkt voranstehenden Anspruch für ein Instrumentenhandstück (1) der Ansprüche 5 bis 9, **dadurch gekennzeichnet, dass** Stromlinien eines Reinigungsfluides, welche vorzugsweise durch das proximale Wälzlager (22) laufen, solche Stromlinien umfassen, welche entlang einer äußeren Mantelfläche des durchgehend ausgebildeten Lagerkäfigs (50) und/oder durch das zumindest eine distale Wälzlager (20) laufen.

## Claims

1. A surgical instrument handpiece (1) for a surgical instrument, comprising:
- a handle section (7) for proximal handling by an operator, and
- a shaft section (8) which extends from the handle section (7) in a distal longitudinal direction, wherein the shaft section (8) is configured with a distal outlet opening (40) at the distal end of the shaft section (8) opposite the handle section (7), to allow a tool that can be replaced by a user to be replaceably arranged in the inner tool holder (19) by inserting it into the distal exit opening (40) of the shaft section (8),
wherein the shaft section (8) has at least one narrowed distal tip section (10) with a reduced cross-sectional area in the region of the distal outlet opening (40), wherein
wherein the shaft section (8) comprises an inner roller bearing configured to rotatably mount the replaceably arrangeable tool, preferably at least in part in the region of the narrowed distal tip section (10), wherein the roller bearing arrangement comprises at least one distal roller bearing (20) and at least one proximal roller bearing (22), and wherein the at least one distal roller bearing (20) and the at least one proximal roller bearing (22) are arranged in a spaced apart manner by means of a bearing cage (50) continuously formed between them,
**characterized in that** the shaft section (8) is configured with an inner tool holder (19) at the other, proximal end of the shaft section.

2. The surgical instrument handpiece (1) according to claim 1, wherein a first diameter (D1) of the narrowed distal tip section (10):
- is smaller in comparison with a second diameter (D2) of a non-narrowed region of the shaft section (8) by a diameter relation factor of a maximum of 95 percent, preferably of a maximum of 85 percent, further preferred of approximately 79 percent; and/or
- amounts to between 3.5 and 5.3 millimeter, preferably to between 4.0 and 5.0 millimeter, further preferred to between 4.3 and 4.5 millimeter.

3. The surgical instrument handpiece (1) according to claim 1 or 2, wherein a first length (L1) of the distal tip section (10):
- is of a length proportion in relation to an entire second length (L2) of the shaft section (8) of at least 5 percent, preferably of at least 20 percent, further preferred of at least 35 percent; and/or
- amounts to between 5 and 40 millimeter, preferably to between 10 and 30 millimeter, further preferred to between 18 and 22 millimeter.

4. The surgical instrument handpiece (1) according to one of the preceding claims, wherein a transition region (11) designed as a shoulder from the narrowed distal tip section (10) to the non-narrowed region of the shaft section (8) is formed in a rounded shape and/or in a gradually tapering off shape and/or in a chamfered shape.

5. The surgical instrument handpiece (1) according to one of the preceding claims, wherein the bearing cage (50) is completely closed or is permeable to a fluid in a small area-related hole volume fraction, wherein the hole volume fraction preferably amounts to less than 40 percent, further preferred to less than 15 percent, particularly preferred to less than 8 percent.

6. The surgical instrument handpiece (1) according to one of the preceding claims, wherein the at least one distal roller bearing (20) and/or the at least one proximal roller bearing (22), preferably all roller bearings of the inner roller bearing arrangement, comprise non-spherical rolling elements and are preferably formed as cylindrical roller bearings and/or as needle bearings.

7. The surgical instrument handpiece (1) according to one of the preceding claims, wherein the at least one distal roller bearing (20) and/or the at least one proximal roller bearing (22), preferably all roller bearings of the inner roller bearing arrangement, comprise ceramic rolling elements.

8. The surgical instrument handpiece (1) according to one of the preceding claims, wherein a bearing cage ring gap cross-sectional area (A-50) of a ring gap arranged in the narrowed distal tip section (10), wherein said ring gap is formed at an outer lateral surface of the continuously formed bearing cage (50) between an inner surface section diameter (d-33) of the inner surface section (33) as a ring gap outer diameter and a bearing cage outer diameter (d-50) as a ring gap inner diameter:
- amounts to less than or equal to 3.5 mm², further preferred to less than or equal to approximately 3 mm² and in particular preferred to less than or equal to 2.8 mm², and/or
- is less than or equal to a flow cross-sectional area through which a free through-flow is possible in a region of the instrument handpiece (1) being proximal thereto, and further preferred is less than or equal to a proximal shaft section ring gap cross-sectional area (A-R) of the shaft section (8).

9. A surgical instrument with an instrument handpiece (1) according to one of the preceding claims, and with a preferably rotatably driven and/or drivable tool.

10. A medical product set, preferably an application-oriented assortment for an operator like a surgeon, having at least one first instrument handpiece (1) according to one of the preceding claims directed to the instrument handpiece (1), in combination with at least:
- one surgical instrument according to the directly preceding claim; and/or
- with a plurality of different medical tools, in particular of rotatably driven and/or drivable medical tools, preferably in an assortment of the tools of different functions and types, and/or of a straight and/or curved shape and/or according to different sizes and/or hardness and/or materials; and/or
- a second instrument handpiece (1) according to one of the preceding claims directed to the instrument handpiece (1), wherein the first instrument handpiece (1) and the second instrument handpiece (2) have different first diameters (D1) and/or different second diameters (D2) and/or different first lengths (L1) and/or different second lengths (D2); and/or
- a tool wrench for the insertion of a corresponding tool into the instrument handpiece (1).

11. A cleaning method for the internal flushing of an instrument handpiece (1) according to the invention according to one of the preceding claims directed to the instrument handpiece (1), in a flow direction from proximal to distal in a rinsing device.

12. The cleaning method according to the directly preceding claim for an instrument handpiece (1) of claims 5 to 9, **characterized in that** flow lines of a cleaning fluid running preferably through the proximal roller bearing (22) include such flow lines which run along the outer lateral surface of the continuously formed bearing cage (50) and/or through the at least one distal roller bearing (20).

## Revendications

1. Pièce à main d'instrument chirurgical (1) pour un instrument chirurgical, comprenant :
- une section de préhension (7) pour la manipulation proximale par un opérateur, et
- une section de tige (8) qui s'étend en direction longitudinale distale à partir de la section de préhension (7), dans laquelle la section de tige (8) est configurée avec une ouverture de sortie distale (40) à l'extrémité distale de la section de tige (8) opposée à la section de préhension (7), pour disposer un outil pouvant être disposé de manière remplaçable par un utilisateur par insertion dans l'ouverture de sortie distale (40) de la section de tige (8) dans le logement d'outil intérieur (19),
dans laquelle la section de tige (8) présente, au niveau de l'ouverture de sortie distale (40), au moins une section de pointe distale (10) à section transversale rétrécie,
dans laquelle la section de tige (8) comprend un roulement intérieur qui est configuré pour supporter de manière rotative l'outil pouvant être disposé de manière remplaçable, de préférence au moins partiellement au niveau de la section de pointe distale rétrécie (10),
dans laquelle le roulement comprend au moins un roulement distal (20) et au moins un roulement proximal (22) et dans laquelle l'au moins un roulement distal (20) et l'au moins un roulement proximal (22) sont espacés l'un de l'autre par une cage de roulement (50) formée de manière continue entre eux,
**caractérisée en ce que** la section de tige (8) est configurée avec un logement d'outil intérieur (19) à l'autre extrémité proximale de la section de tige.

2. Pièce à main d'instrument chirurgical (1) selon la revendication 1, dans laquelle un premier diamètre (D1) de la section distale rétrécie (10) :
- est plus petit par rapport à un second diamètre (D2) d'une zone non rétrécie de la section de tige (8) d'un facteur de relation de diamètre maximal de 95 pour cent, de préférence maximal de 85 pour cent, plus préférablement d'env. 79 pour cent ; et/ou
- est compris entre 3,5 et 5,3 millimètres, de préférence entre 4,0 et 5,0 millimètres, plus préférablement entre 4,3 et 4,5 millimètres.

3. Pièce à main d'instrument chirurgical (1) selon la revendication 1 ou 2, dans laquelle une première longueur (L1) de la section de pointe distale (10) :
- par rapport à une seconde longueur totale (L2) de la section de tige (8), représente une proportion de longueur d'au moins 5 pour cent, de préférence d'au moins 20 pour cent, plus préférablement d'au moins 35 pour cent ; et/ou
- est compris entre 5 et 40 millimètres, de préférence entre 10 et 30 millimètres, plus préférablement entre 18 et 22 millimètres.

4. Pièce à main d'instrument chirurgical (1) selon l'une quelconque des revendications précédentes, dans laquelle une zone de transition (11) formée en tant que décrochement entre la section distale rétrécie (10) et la zone non rétrécie de la section de tige (8) est arrondie et/ou s'étend de manière graduelle et/ou est biseautée.

5. Pièce à main d'instrument chirurgical (1) selon l'une quelconque des revendications précédentes, dans laquelle la cage de roulement (50) est entièrement fermée ou perméable aux fluides avec une faible proportion de volume de trous par rapport à la surface, dans laquelle la proportion de volume de trous est de préférence inférieure à 40 pour cent, plus préférablement inférieure à 15 pour cent, et en particulier de préférence inférieure à 8 pour cent.

6. Pièce à main d'instrument chirurgical (1) selon l'une quelconque des revendications précédentes, dans laquelle au moins un roulement distal (20) et/ou au moins un roulement proximal (22), de préférence tous les roulements du roulement intérieur, présentent des corps de roulement non sphériques, de préférence en tant que roulements à rouleaux cylindriques et/ou de roulements à aiguilles.

7. Pièce à main d'instrument chirurgical (1) selon l'une quelconque des revendications précédentes, dans laquelle au moins un roulement distal (20) et/ou au moins un roulement proximal (22), de préférence tous les roulements du roulement intérieur, présentent des corps de roulement céramiques.

8. Pièce à main d'instrument chirurgical (1) selon l'une quelconque des revendications précédentes, dans laquelle une surface transversale d'espace annulaire de cage de roulement (A-50), laquelle est disposée dans la section de pointe distale rétrécie (10), est formée sur la surface d'enveloppe extérieure de la cage de roulement (50) formée de manière continue, entre un diamètre de section de surface intérieure (d-33) de la section de surface intérieure (33) en tant que diamètre extérieur d'espace annulaire et un diamètre extérieur (d-50) de la cage de roulement en tant que diamètre intérieur d'espace annulaire :
- inférieure ou égale à 3,5 mm², de préférence inférieure ou égale à env. 3 mm² et en particulier de préférence inférieure ou égale à 2,8 mm² ; et/ou
- inférieure ou égale à une surface de section transversale d'écoulement, laquelle peut être traversée librement dans une zone proximale de la pièce à main d'instrument (1), de préférence inférieure ou égale à une surface de section transversale d'espace annulaire de section de tige proximale (A-R) de la section de tige proximale (8).

9. Instrument chirurgical avec une pièce à main d'instrument (1) selon l'une quelconque des revendications précédentes et un outil, de préférence entraîné et/ou entraînable de manière rotative.

10. Ensemble de produits médicaux, de préférence une combinaison adaptée à une application pour un opérateur tel qu'un chirurgien, avec au moins une première pièce à main d'instrument (1) selon l'une quelconque des revendications précédentes, orientée vers la pièce à main d'instrument (1), en combinaison avec au moins :
- un instrument chirurgical selon la revendication immédiatement précédente ; et/ou
- avec une pluralité d'outils médicaux différents, en particulier entraînés et/ou pouvant être entraînés de manière rotative, de préférence dans une combinaison d'outils de fonctions différentes et/ou de forme droite et/ou courbée et/ou de tailles et/ou de degrés de dureté et/ou de matériaux différents ; et/ou
- une seconde pièce à main d'instrument (1) selon l'une quelconque des revendications précédentes, orientée vers la pièce à main d'instrument (1), dans laquelle la première pièce à main d'instrument (1) et la seconde pièce à main d'instrument (2) présentent de premiers diamètres (D1) différents et/ou de seconds diamètres (D2) différents et/ou de premières longueurs (L1) différentes et/ou de secondes longueurs (D2) différentes ; et/ou
- une clé à outil pour insérer un outil correspondant dans la pièce à main d'instrument (1).

11. Procédé de nettoyage pour le rinçage intérieur d'une pièce à main d'instrument (1) conforme à l'invention selon l'une quelconque des revendications précédentes, dirigé vers la pièce à main d'instrument (1), dans le sens d'écoulement proximal à distal dans un dispositif de rinçage.

12. Procédé de nettoyage selon la revendication directement précédente pour une pièce à main d'instrument (1) des revendications 5 à 9, **caractérisé en ce que** des lignes de courant d'un fluide de nettoyage, lesquelles passent de préférence par le roulement proximal (22), comprennent des lignes de courant, lesquelles passent le long d'une surface d'enveloppe extérieure de la cage de roulement (50) conçue de manière continue et/ou par l'au moins un roulement distal (20).
